# EUROPEAN PATENT APPLICATION

(11) **EP 4 552 649 A2**
(43) Date of publication of application: **14.05.2025**
(21) Application number: 25167129.3
(22) Date of filing: 28.08.2020
(51) Int. Cl.: A61K 45/06

(54) **THERAPY GUIDANCE AND/OR THERAPY MONITORING FOR TREATMENT OF SHOCK**

(30) Priority: 30.08.2019 EP 19194729; 02.10.2019 EP 19201098
(62) Divisional of application: 20764639.9
(71) Applicant: 4TEEN4 Pharmaceuticals GmbH, 16761 Hennigsdorf (DE)
(72) Inventor: Bergmann, Andreas, 13465 Berlin (DE)
(74) Representative: Kilger, Ute

(57) **Abstract**

Subject matter of the present invention is a method for predicting or diagnosing a refractory shock in a subject that either runs into shock or that has developed shock, wherein said method is comprising the steps:
• determining the level of DPP3 in a sample of bodily fluid of said subject;
• comparing said level of determined DPP3 to a predetermined threshold,
wherein said subject is predicted to run into refractory shock or is diagnosed as having refractory shock if said determined level of DPP3 is above said predetermined threshold.

Further subject matter relates to vasopressors, angiotensin-receptor agonists and/or precursors thereof, inhibitors of the activity of DPP3 and anti-ADM antibodies for use in therapy of shock in a subject that either runs into shock or that has developed shock.

## Description

The present invention is directed to a method for predicting or diagnosing a refractory shock in a subject that either runs into shock or that has developed shock, and vasopressors, angiotensin-receptor agonists and/or precursors thereof, inhibitors of DPP3 and anti-ADM antibodies for use in therapy of shock in a subject that either runs into shock or that has developed shock, and methods of treating said shock or refractory shock.

### STATE OF THE ART

Dipeptidyl peptidase 3 - also known as Dipeptidyl aminopeptidase III, Dipeptidyl arylamidase III, Dipeptidyl peptidase III, Enkephalinase B or red cell angiotensinase; short name: DPP3, DPPIII - is a metallopeptidase that removes dipeptides from physiologically active peptides, such as enkephalins and angiotensins.

DPP3 was first identified and its activity measured in extracts of purified bovine anterior pituitary by Ellis & Nuenke 1967. The enzyme, which is listed as EC 3.4.14.4, has a molecular mass of about 83 kDa and is highly conserved in procaryotes and eucaryotes (*Prajapati & Chauhan 2011*). The amino acid sequence of the human variant is depicted in SEQ ID NO 1. Dipeptidyl peptidase III is a mainly cytosolic peptidase which is ubiquitously expressed. Despite lacking a signal sequence, a few studies reported membranous activity

### (Lee & Snyder 1982).

DPP3 is a zinc-depending exo-peptidase belonging to the peptidase family M49. It has a broad substrate specificity for oligopeptides from three/ four to ten amino acids of various compositions and is also capable of cleaving after proline. DPP3 is known to hydrolyze dipeptides from the N-terminus of its substrates, including angiotensin II, III and IV; Leu- and Met-enkephalin; endomorphin 1 and 2. The metallopeptidase DPP3 has its activity optimum at pH 8.0-9.0 and can be activated by addition of divalent metal ions, such as Co²⁺ and Mg²⁺.

Structural analysis of DPP3 revealed the catalytic motifs HELLGH (hDPP3 450-455) and EECRAE (hDPP3 507-512), as well as following amino acids, that are important for substrate binding and hydrolysis: Glu316, Tyr, 318, Asp366, Asn391, Asn394, His568, Arg572, Arg577, Lys666 and Arg669 (*Prajapati & Chauhan 2011; Kumar et al. 2016;* numbering refers to the sequence of human DPP3, see SEQ ID NO. 1). Considering all known amino acids or sequence regions that are involved in substrate binding and hydrolysis, the active site of human DPP3 can be defined as the area between amino acids 316 and 669.

The most prominent substrate of DPP3 is angiotensin II (Ang II), the main effector of the renin-angiotensin system (RAS). The RAS is activated in cardiovascular diseases (Dostal et al. 1997. J Mol Cell Cardiol;29:2893-902*;* Roks et al. 1997. Heart Vessels. Suppl 12:119-24), sepsis, and septic shock (Corrêa et al. 2015. Crit Care 2015;19:98*).* Ang II, in particular, has been shown to modulate many cardiovascular functions including the control of blood pressure and cardiac remodeling.

The exact biological function of DPP3 in cellular physiology is not understood, but recent findings indicate its role not only in in protein metabolism but also in pain modulation and inflammatory processes (*Prajapati & Chauhan 2011).* In addition, DPP3 reduced blood pressure in AngII-infused hypertensive mice, without changing heart rate (*Pang et al. 2016).* However normotensive mice had no alteration in blood pressure, when DPP3 was injected.

Blood pressure reduction by administration of DPP3 together with an angiotensin-receptor antagonist in Ang II-infused mice was similar to that of DPP3 injection alone or angiotensin-receptor antagonist alone *(*Pang et al. 2016. Hypertension 68:630-41*).*

Angiotensin II (AngII) is a peptide hormone naturally produced by the body that regulates blood pressure via vasoconstriction and sodium reabsorption. The hemodynamic effects of Ang II administration have been the subject of numerous clinical studies, demonstrating significant effects on systemic and renal blood flow *(*Harrison-Bernard 2009. Adv. Physiol Edu. 33(4): 270*).*

AngII-treatment is currently discussed for its beneficial effect in vasodilatory shock and septic shock (*Khanna. A. et al., 2017; Antonucci, E. et al. 2017, Tumlin, J.A. et al. 2018).* Patients with vasodilatory shock (80% in septic shock) treated with angiotensin II were more likely to survive to 28 days and showed a significant correction of hypotension (*Khanna, A. et al.,*

### 2017; Tumlin, J.A. et al. 2018).

It is assumed that the Angiotensin-converting enzyme (ACE) is highly dysregulated in shock patients leading to an altered angiotensin I/II ratio) and that angiotensin II infusion may compensate for such dysregulation *(Tumlin, J.A. et al. 2018).*

Recently, two assays were generated, characterized, and validated to specifically detect DPP3 in human bodily fluids (e.g., blood, plasma, serum): a luminescence immunoassay (LIA) to detect DPP3 protein concentration and an enzyme capture activity assay (ECA) to detect specific DPP3 activity *(*Rehfeld et al. 2019 JALM 3(6):943-953*).* A washing step in both methods removes all interfering substances before the actual detection of DPP3 protein or activity is performed. Both methods are highly specific and allow the reproducible detection of DPP3 in blood samples.

The object of the present invention is the provision of a method for predicting or diagnosing a refractory shock in a subject that either runs into shock or that has developed shock.

Another object of the invention is a provision of Vasopressors, angiotensin-receptor agonists and/or precursors thereof, inhibitors of DPP3 and anti-ADM antibodies for use in therapy of shock in a subject that either runs into shock or that has developed shock.

Subject of the present invention is a method for predicting or diagnosing a refractory shock in a subject that either runs into shock or that has developed shock, wherein said method is comprising the steps:
- determining the level of DPP3 in a sample of bodily fluid of said subject;
- comparing said level of determined DPP3 to a predetermined threshold,
wherein said subject is predicted to run into refractory shock or is diagnosed as having refractory shock if said determined level of DPP3 is above said predetermined threshold.

Subject of the present invention is a method for predicting or diagnosing a refractory shock in a subject that either runs into shock or that has developed shock, wherein said method is comprising the steps:
- determining the level of DPP3 in a sample of bodily fluid of said subject, and
- comparing said level of determined DPP3 to a predetermined threshold, and
- determining the level of pro-adrenomedullin of fragments thereof in a sample of bodily fluid of said subject, and
- comparing said level of determined pro-adrenomedullin or fragments thereof to a predetermined threshold,
wherein said subject is predicted to run into refractory shock or is diagnosed as having refractory shock if said determined level of pro-adrenomedullin or fragments thereof and/or said level of DPP3 is above said predetermined threshold.

In a specific embodiment of said method for predicting or diagnosing a refractory shock in a subject that either runs into shock or that has developed shock, said shock is selected from the group comprising shock due to hypovolemia, cardiogenic shock, obstructive shock and distributive shock.

**In** another specific embodiment of said method for predicting or diagnosing a refractory shock in a subject that either runs into shock or that has developed shock, said shock is selected from the group comprising shock due to hypovolemia, cardiogenic shock, obstructive shock and distributive shock, in particular cardiogenic or septic shock.

**In** a specific embodiment of said method for predicting or diagnosing a refractory shock in a subject that either runs into shock or that has developed shock, said shock is selected from the group comprising:
- in case of cardiogenic shock said subject has suffered an acute coronary syndrome (e.g. acute myocardial infarction) or has heart failure (e.g. acute decompensated heart failure), myocarditis, arrhythmia, cardiomypathy, valcular heart disease, aortic dissection with acute aortic stenosis, traumatic chordal rupture or massive pulmonary embolism, or
- in case of hypovolemic shock said subject may have suffered a hemorrhagic disease including gastrointestinal bleed, trauma, vascular etiologies (e.g. ruptured abdominal aortic aneurysm, tumor eroding into a major blood vessel) and spontaneous bleeding in the setting of anticoagulant use or a non-hemorrhagic disease including vomiting, diarrhea, renal loss, skin losses/insensible losses (e.g. burns, heat stroke) or third-space loss in the setting of pancreatitis, cirrhosis, intestinal obstruction, trauma, or
- in case of obstructive shock said patient may have suffered a cardiac tamponade, tension pneumothorax, pulmonary embolism or aortic stenosis, or
- in case of distributive shock said patient has septic shock, neurogenic shock, anaphylactic shock or shock due to adrenal crisis.

**In** another embodiment of said method for predicting or diagnosing a refractory shock in a subject that either runs into shock or that has developed shock, said method is used for initiation and/or termination and/ or stratification and/or guidance of treatment.

As used herein, the term "therapy guidance" or "guidance of treatment" refers to application of certain therapies or medical interventions based on the value of one or more biomarkers and/or clinical parameter and/or clinical scores.

The term "therapy monitoring" in the context of the present invention refers to the monitoring and/or adjustment of a therapeutic treatment of said patient, for example by obtaining feedback on the efficacy of the therapy.

The term "therapy stratification" in particular relates to grouping or classifying patients into different groups, such as therapy groups that receive or do not receive therapeutic measures depending on their classification.

The term "prediction" means correlating a probability of an outcome with a result obtained in the measurement of an analyte. An example of this is the measurement of a certain marker, such as DPP3, in a sample, the measured level of which is correlated with the probability of a subject that either runs into shock or that has developed shock, to develop a refractory shock.

The present invention also comprises a method for short-term prediction of a refractory shock in a subject that either runs into shock or that has developed shock, whereby short-term means a period of equal or less than 14 days, preferably equal or less than 7 days, more preferred equal or less than 3 days, most preferred equal or less than 48 hours.

In another embodiment of said method for predicting or diagnosing a refractory shock in a subject that either runs into shock or that has developed shock, said treatment is initiated and/or maintained and/or withheld and/or terminated if said determined level of DPP3 is above said predetermined threshold.

In another embodiment of said method for predicting or diagnosing a refractory shock in a subject that either runs into shock or that has developed shock, said treatment is initiated and/or maintained and/or withheld and/or terminated if said determined level of DPP3 and/ or pro-adrenomedullin and/ or fragments thereof is above said predetermined threshold.

In a preferred embodiment of said method for predicting or diagnosing a refractory shock in a subject that either runs into shock or that has developed shock, said treatment is selected from the group of vasopressors, angiotensin receptor agonists or precursors thereof, and/or inhibitors of the DPP3 activity.

In another preferred embodiment of said method for predicting or diagnosing a refractory shock in a subject that either runs into shock or that has developed shock, said treatment is selected from the group of vasopressors, Angiotensin-Receptor-Agonists and/or precursors thereof, inhibitors of the DPP3 activity and anti-adrenomedullin antibodies or anti-adrenomedullin antibody fragments.

In another embodiment of said method for predicting or diagnosing a refractory shock in a subject that either runs into shock or that has developed shock either the level of DPP3 protein and/or the level of active DPP3 is determined and compared to a predetermined threshold.

In another embodiment of said method for predicting or diagnosing a refractory shock in a subject that either runs into shock or that has developed shock the level of DPP3 is determined by contacting said sample of bodily fluid with a capture binder that binds specifically to DPP3.

In another preferred embodiment of said method for predicting or diagnosing a refractory shock in a subject that either runs into shock or that has developed shock, said capture binder for determining the level of DPP3 may be selected from the group of antibody, antibody fragment or non-IgG scaffold.

In a specific embodiment of said method method for predicting or diagnosing a refractory shock in a subject that either runs into shock or that has developed shock, said capture binder is an antibody.

In another specific embodiment of said method for predicting or diagnosing a refractory shock in a subject that either runs into shock or that has developed shock, said capture binder is a monoclonal antibody.

In another embodiment of said method for predicting or diagnosing a refractory shock in a subject that either runs into shock or that has developed shock, said sample of bodily fluid is selected from the group of whole blood, plasma, and serum.

In a specific embodiment of said method for predicting or diagnosing a refractory shock in a subject that either runs into shock or that has developed shock, said method of diagnosing or predicting is conducted at least twice.

Shock is characterized by decreased oxygen delivery and/or increased oxygen consumption or inadequate oxygen utilization leading to cellular and tissue hypoxia. It is a life-threatening condition of circulatory failure and most commonly manifested as hypotension (systolic blood pressure less than 90 mm Hg or MAP less than 65 mmHg). Shock is divided into four main types based on the underlying cause: hypovolemic, cardiogenic, obstructive, and distributive shock *(*Vincent and De Backer 2014. N. Engl. J. Med. 370(6): 583*).*

Hypovolemic shock is characterized by decreased intravascular volume and can be divided into two broad subtypes: hemorrhagic and nonhemorrhagic. Common causes of hemorrhagic hypovolemic shock include gastrointestinal bleed, trauma, vascular etiologies (e.g. ruptured abdominal aortic aneurysm, tumor eroding into a major blood vessel) and spontaneous bleeding in the setting of anticoagulant use. Common causes of nonhemorrhagic hypovolemic shock include vomiting, diarrhea, renal loss, skin losses/insensible losses (e.g. burns, heat stroke) or third-space loss in the setting of pancreatitis, cirrhosis, intestinal obstruction, trauma. For review see Koya and Paul 2018. Shock. StatPearls [Internet]. Treasure Island (FL): StatPearls Publishing; 2019-2018 Oct 27*.*

Cardiogenic shock (CS) is defined as a state of critical endorgan hypoperfusion due to reduced cardiac output. Notably, CS forms a spectrum that ranges from mild hypoperfusion to profound shock. Established criteria for the diagnosis of CS are: (i) systolic blood pressure, ≤90 mmHg for >30 min or vasopressors required to achieve a blood pressure ≥90 mmHg; (ii) pulmonary congestion or elevated left-ventricular filling pressures; (iii) signs of impaired organ perfusion with at least one of the following criteria: (a) altered mental status; (b) cold, clammy skin; (c) oliguria (< 0.5 mL/kg/h or <30 mL/h); (d) increased serum-lactate (Reynolds and Hochman 2008. Circulation 117: 686-697)*.* Acute myocardial infarction (AMI) with subsequent ventricular dysfunction is the most frequent cause of CS accounting for approximately 80% of cases. Mechanical complications such as ventricular septal (4%) or free wall rupture (2%), and acute severe mitral regurgitation (7%) are less frequent causes of CS after AMI. *(*Hochman et al. 2000. J Am Coll Cardiol 36: 1063-1070*).* Non-AMI-related CS may be caused by decompensated valvular heart disease, acute myocarditis, arrhythmias, etc. with heterogeneous treatment options. This translates in 40 000 to 50 000 patients per year in the USA and 60 000 to 70 000 in Europe. Despite advances in treatment mainly by early revascularization with subsequent mortality reduction, CS remains the leading cause of death in AMI with mortality rates still approaching 40-50% according to recent registries and randomized trials *(*Goldberg et al. 2009. Circulation 119: 1211-1219*).*

Obstructive shock is due to a physical obstruction of the great vessels or the heart itself. Several conditions can result in this form of shock (e.g. cardiac tamponade, tension pneumothorax, pulmonary embolism, aortic stenosis). For review see Koya and Paul 2018. Shock. StatPearls [Internet]. Treasure Island (FL): StatPearls Publishing; 2019-2018 Oct 27*.*

According to the cause, there are four types of distributive shock: neurogenic shock (decreased sympathetic stimulation leading to decreased vasal tone), anaphylactic shock, septic shock and shock due to adrenal crisis. In addition to sepsis, distributive shock can be caused by systemic inflammatory response syndrome (SIRS) due to conditions other than infection such as pancreatitis, burns or trauma. Other causes include, toxic shock syndrome (TSS), anaphylaxis (a sudden, severe allergic reaction), adrenal insufficiency (acute worsening of chronic adrenal insufficiency, destruction or removal of the adrenal glands, suppression of adrenal gland function due to exogenous steroids, hypopituitarism and metabolic failure of hormone production), reactions to drugs or toxins, heavy metal poisoning, hepatic (liver) insufficiency and damage to the central nervous system. For review see Koya and Paul 2018. Shock. StatPearls [Internet]. Treasure Island (FL): StatPearls Publishing; 2019-2018 Oct 27*.*

In general, refractory shock has been defined as requirement of noradrenaline infusion of >0.5 µg/kg/min despite adequate volume resuscitation. Mortality in these patients may be as high as 94% and the assessment and management of these patients requires a much more aggressive approach for survival. The term "refractory shock" is used when the tissue perfusion cannot be restored with the initial corrective measures employed (e.g. vasopressors) and may therefore be referred to as "high vasopressor-dependent" or "vasopressor-resistant" shock (Udupa and Shetty 2018. Indian J Respir Care 7: 67□72). Patients with refractory shock may have features of inadequate perfusion such as hypotension (mean arterial blood pressure <65 mmHg), tachycardia, cold peripheries, prolonged capillary refill time, and tachypnea consequent to the hypoxia and acidosis. Fever may be seen in septic shock. Other signs of hypoperfusion such as altered sensorium, hyperlactatemia, and oliguria may also be seen. These well 0 known signs of shock are not helpful in identifying whether the problem is at the pump (heart) or circuitry (vessels and tissues). Different types of shock can coexist, and all forms of shock can become refractory, as evidenced by unresponsiveness to high□dose vasopressors (Udupa and Shetty 2018. Indian J Respir Care 7: 67□72)*.*

In a specific embodiment of the invention said refractory shock is vasopressor-resistant, that is defined by unresponsiveness of the patient to high dose vasopressors (e.g. >0.5 µg/kg/min noradrenaline).

The consequence of the diagnosis or the prediction that a subject either runs into a vasopressor-resistant refractory shock or is diagnosed with a vasopressor-resistant refractory shock maybe the administration of treatment like administration of angiotensin II inhibitors and/or DPP3 inhibitors. Furthermore, another consequence maybe the withholding of vasopressors.

In a preferred embodiment of said method for predicting a refractory shock, in particular a vasopressor-resitant shock, in a subject that potentially runs into shock, wherein it is predicted whether said subject runs into a refractory shock, in particular a vasopressor-resitant shock, from the point of time the sample is taken within 14 days, or in particular within 10 days, or in particular within 7 days, or in particular within 5 days, or in particular within 48 hours, or in particular within 24 hours, or in particular between 24 and 48 hours.

Another embodiment of the present invention relates to a method for predicting or diagnosing a refractory shock in a subject that either runs into shock or that has developed shock, wherein a treatment with Angiotensin-Receptor-Agonists and/or precursors thereof and/or inhibitors of the DPP3 activity is initiated and/or continued when the level of DPP3 in said sample is above a certain threshold and/or wherein a treatment with vasopressors is withheld and/ or terminated if said determined level of DPP3 is above said predetermined threshold.

Another specific embodiment of the present invention relates to a method for predicting or diagnosing a refractory shock in a subject that either runs into shock or that has developed shock, wherein a treatment with vasopressors is initiated and/or continued when the level of DPP3 in said sample is below a certain threshold and/or wherein a treatment with Angiotensin-Receptor-Agonists and/or precursors thereof and/or inhibitors of the DPP3 activity is withheld and/ or terminated if the said determined level of DPP3 is below said predetermined threshold.

A further embodiment of the present invention relates to a method for predicting or diagnosing a refractory shock in a subject that either runs into shock or that has developed shock, wherein a treatment with vasopressors is initiated and/or continued when the level of DPP3 in said sample is below a certain threshold and/or wherein a treatment with Angiotensin-Receptor-Agonists and/or precursors thereof and/or inhibitors of the DPP3 activity is withheld and/ or terminated if the said determined level of DPP3 is below said predetermined threshold and the level of Pro-adrenomedullin or fragments thereof is determined and wherein treatment with an anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

A further embodiment of the present invention relates to a method for predicting or diagnosing a refractory shock in a subject that either runs into shock or that has developed shock according to the present invention, wherein
- said subject may have suffered an acute coronary syndrome (e.g. acute myocardial infarction) or wherein said subject has heart failure (e.g. acute decompensated heart failure), myocarditis, arrhythmia, cardiomyopathy, valvular heart disease, aortic dissection with acute aortic stenosis, traumatic chordal rupture or massive pulmonary embolism (in case of cardiogenic shock), or
- said subject may have suffered a hemorrhagic disease including gastrointestinal bleed, trauma, vascular etiologies (e.g. ruptured abdominal aortic aneurysm, tumor eroding into a major blood vessel) and spontaneous bleeding in the setting of anticoagulant use or a non-hemorrhagic disease including vomiting, diarrhea, renal loss, skin losses/insensible losses (e.g. burns, heat stroke) or third-space loss in the setting of pancreatitis, cirrhosis, intestinal obstruction, trauma, (in case of hypovolemic shock) or
- said patient may have suffered a cardiac tamponade, tension pneumothorax, pulmonary embolism or aortic stenosis (in case of obstructive shock), or
- said patient may have septic shock, neurogenic shock, anaphylactic shock or shock due to adrenal crisis (in case of distributive shock.)

A further embodiment of the present invention relates to a method for predicting or diagnosing a refractory shock in a subject that either runs into shock or that has developed shock according to the present invention, wherein said method is used for initiation and/or termination and/ or stratification and/or guidance of treatment.

A further embodiment of the present invention relates to a method for predicting or diagnosing a refractory shock in a subject that either runs into shock or that has developed shock according to the present invention, wherein a treatment is initiated and/or maintained and/or withheld and/ or terminated if said determined level of DPP3 is above said predetermined threshold.

Subject matter of the present invention are Vasopressors, Angiotensin-Receptor-Agonists and/or precursors thereof, inhibitors of the DPP3 activity and/or anti-adrenomedullin antibodies or anti-adrenomedullin antibody fragments for use in a method of prevention or treatment of a refractory shock in a subject that either either runs into shock or that has developed shock, wherein said prediction and/or diagnosis of said refractory shock has been determined by a method for predicting or diagnosing a refractory shock in a subject that either runs into shock or that has developed shock according to the present invention. In one embodiment of the beforementioned methods either the level of DPP3 protein and/or the level of active DPP3 is determined and compared to a predetermined threshold, wherein the level of DPP3 is determined by contacting said sample of bodily fluid with a capture binder that binds specifically to DPP3.

Subject matter of the present invention are Angiotensin-Receptor-Agonists and/or precursors thereof, inhibitors of the DPP3 activity for use in a method of prevention or treatment of a refractory shock in a subject that either either runs into shock or that has developed shock, wherein said prediction and/or diagnosis of said refractory shock has been determined by a method for predicting or diagnosing a refractory shock in a subject that either runs into shock or that has developed shock according to the present invention, wherein a treatment with Angiotensin-Receptor-Agonists and/ or precursors thereof and/or inhibitors of the DPP3 activity is initiated and/or continued when the level of DPP3 in said sample is above a certain threshold and/or wherein a treatment with vasopressors is withheld and/ or terminated if said determined level of DPP3 is above said predetermined threshold.

Subject matter of the present invention are Angiotensin-Receptor-Agonists and/or precursors thereof, inhibitors of the DPP3 activity for use in a method of prevention or treatment of a refractory shock in a subject that either either runs into shock or that has developed shock, wherein said prediction and/or diagnosis of said refractory shock has been determined by a method for predicting or diagnosing a refractory shock in a subject that either runs into shock or that has developed shock according to the present invention, wherein a treatment with vasopressors is initiated and/or continued when the level of DPP3 in said sample is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.

Subject matter of the present invention is an anti-ADM antibody or anti-ADM antibody fragment for use in a method of prevention or treatment of a refractory shock in a subject that either either runs into shock or that has developed shock, wherein said prediction and/or diagnosis of said refractory shock has been determined by a method for predicting or diagnosing a refractory shock in a subject that either runs into shock or that has developed shock according to the present invention, wherein in addition the level of Pro-adrenomedullin or fragments thereof is determined and wherein treatment with an anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

Another embodiment of the present invention relates to a method for predicting or diagnosing a refractory shock in a subject that either runs into shock or that has developed shock, wherein treatment with an anti-ADM antibody or anti-ADM antibody fragment and/or Angiotensin-Receptor-Agonists and/or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.

Subject matter of the present invention is an anti-ADM antibody or anti-ADM antibody fragment and/or an inhibitor of the DPP3 activity for use in a method of prevention or treatment of a refractory shock in a subject that either either runs into shock or that has developed shock, wherein said prediction and/or diagnosis of said refractory shock has been determined by a method for predicting or diagnosing a refractory shock in a subject that either runs into shock or that has developed shock according to the present invention, wherein treatment with an anti-ADM antibody or anti-ADM antibody fragment and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.

In a specific embodiment of the invention the threshold is within a threshold range for plasma DPP3 that is between 20 and 200 ng/ml, preferably between 25 and 150 ng/ml, even more preferred between 30 and 100 ng/ml, even more preferred between 35 and 75 ng/ml, most preferred a threshold of 50 ng/mL is applied.

In particular the refractory shock of said patient is a vasopressor-resistant shock.

The peptide adrenomedullin (ADM) was described as a novel hypotensive peptide comprising 52 amino acids, which had been isolated from a human pheochromocytoma *(*Kitamura et al. 1993. Biochemical and Biophysical Research Communications 192 (2): 553-560*).* Cleavage of the N-terminal signal sequence of 21 amino acids from pre-pro-Adrenomedullin (pre-proADM) results in the precursor peptide pro-Adrenomedullin (proADM) (SEQ ID No.: 31). Pro-ADM is further cleaved into PAMP (SEQ ID No. 32), MR-proADM (SEQ ID No. 33), ADM-Gly (SEQ ID No.: 35) and CT-proADM (SEQ ID No. 36). The mature adrenomedullin peptide is an amidated peptide (ADM-NH₂), which comprises 52 amino acids (SEQ ID No: 34) and which comprises the amino acids 95 to 146 of pre-proADM, from which it is formed by proteolytic cleavage. Mature ADM, bio-ADM and ADM-NH₂ is used synonymously throughout this application and is a molecule according to SEQ ID No.: 34.

In one embodiment of the subject matter of the present invention said fragment of pro-Adrenomedullin is selected from a group comprising PAMP (SEQ ID No. 32), MR-proADM (SEQ ID No. 33), amidated ADM (SEQ ID No. 34), ADM-Gly (SEQ ID No.: 35) and CT-proADM (SEQ ID No. 36).

ADM may be regarded as a polyfunctional regulatory peptide. It is released into the circulation partially in an inactive form extended by glycine *(*Kitamura et al. 1998. Biochem. Biophys. Res. Commun. 244(2): 551-555). There is also a binding protein *(*Pio et al. 2001. The Journal of Biological Chemistry 276(15): 12292-12300*),* which is specific for ADM and probably likewise modulates the effect of ADM.

Those physiological effects of ADM as well as of PAMP, which are of primary importance in the investigations to date, were the effects influencing blood pressure. Thus, ADM is an effective vasodilator. It was found that the concentrations of ADM, which can be measured in the circulation and other biological fluids, are in a number of pathological states, significantly above the concentrations to be found in healthy control persons. Thus, the ADM level in patients with congestive heart failure, myocardial infarction, kidney diseases, hypertensive disorders, diabetes mellitus, in the acute phase of shock and in sepsis and septic shock are significantly increased, although to different extents. The PAMP concentrations are also increased in some of said pathological states, but the plasma levels are reduced relative to ADM *(*Eto et al. 2001. Peptides 22: 1693-1711*).*

Furthermore, it is known that unusual high concentrations of ADM are to be observed in sepsis or in septic shock *(*Eto et al. 2001. Peptides 22: 1693-1711*;* Hirata et al. 1996. Journal of Clinical Endocrinology and Metabolism 81(4): 1449-1453*;* Ehlenz et al. 1997. Exp Clin Endocrinol Diabetes 105: 156-162*;* Tomoda et al. 2001. Peptides 22: 1783-1794*;* Ueda et al. 1999 Am. J. Respir. Crit. Care Med.160: 132-136*;* Wang et al. 2001. Peptides 22: 1835-1840*).* The findings are related to the typical hemodynamic changes which are known as typical phenomena of the course of a disease in patients with sepsis and other severe syndromes, such as, for example, SIRS. Adrenomedullin plays pivotal roles during sepsis development (Wang, Shock 1998, 10(5):383-384*;* Wang et al. 1998. Archives of surgery 133(12): 1298-1304*)* and in numerous acute and chronic diseases (Parlapiano et al. 1999. European Review for Medical and Pharmacological Sciences 3:53-61*;* Hinson et al. 2000 Endocrine Reviews 21(2):138-167)*.*

MR-proADM has been identified as a prognostic marker that is able to stratify the mortality risk in sepsis patients with different degrees of organ failure. It may be helpful in the early identification and individual risk assessment of sepsis and may also facilitate the subsequent clinical management of sepsis and septic shock (for review see Önal et al. 2018. Healthcare 6: 110*).*

Several methods were described to measure circulating levels of ADM: either ADM directly or indirectly by determining a more stable fragment of its cognate precursor peptide. Recently a method was published, describing an assay to measure circulating mature ADM *(*Weber et al. 2017. JALM 2: 222-233).

Other methods to quantify fragments derived from the ADM precursor have been described, e.g. the measurement of MR-proADM (Morgenthaler et al. 2005. Clin Chem 51(10):1823-9), PAMP (Washimine et al. 1994. Biochem Biophys Res Commun 202(2):1081-7*)* and CT-proADM (EP 2 111 552). A commercial homogeneous time-resolved fluoroimmunoassay for the measurement of MR-proADM in plasma on a fully automated system is available (BRAHMS MR-proADM KRYPTOR; BRAHMS GmbH, Hennigsdorf, Germany) *(*Caruhel et al. 2009. Clin Biochem 42(7-8):725-8*).* As these peptides are generated in a stoichiometric ratio from the same precursor, their plasma levels are correlated to a certain extent.

The generation of anti-ADM antibodies is known in the art (e.g. WO2013072512 and WO2019057992 incorporated herein by reference).

Another embodiment of the invention relates to an anti-ADM antibody or an anti-ADM antibody fragment for use in treatment of shock in a subject that either runs into shock or that has developed shock, wherein said antibody or fragment or scaffold binds to the N-terminal part (aa 1-21) of mature ADM: YRQSMNNFQGLRSFGCRFGTC (SEQ ID No. 37).

In another preferred embodiment said anti-ADM antibody or an anti-ADM antibody fragment recognizes and binds to the N-terminal end (aa1) of ADM. N-terminal end means that the amino acid 1, that is "Y" of SEQ ID No. 34 and 35 is mandatory for antibody binding. Said antibody or fragment or non-Ig scaffold would neither bind N-terminal extended nor N-terminally modified ADM nor N-terminally degraded ADM.

Another embodiment of the invention relates to an anti-ADM antibody or an anti-ADM antibody fragment for use in treatment of shock in a subject that either runs into shock or that has developed shock, wherein said antibody or fragment is a human monoclonal antibody or fragment that binds to the N-terminal region (aa 1-21) of mature ADM (SEQ ID No. 37) or an antibody fragment thereof wherein the heavy chain comprises the sequences:
GYTFSRYW (CDR1: SEQ ID NO: 38), ILPGSGST (CDR2: SEQ ID NO: 39), TEGYEYDGFDY (CDR3: SEQ ID NO: 40) and
wherein the light chain comprises the sequences:
   QSIVYSNGNTY (CDR1: SEQ ID NO: 41), RVS (CDR2), FQGSHIPYT (CDR3: SEQ ID NO: 42).

Another embodiment of the invention relates to an anti-ADM antibody or an anti-ADM antibody fragment for use in treatment of shock in a subject that either runs into shock or that has developed shock, wherein said antibody is a monoclonal antibody comprising the following sequence as a heavy chain: 
QVQLVQSGAEVKKPGSSVKVSCKASGYTFSRYWIEWVRQAPGQGLEWIGEILPGSG
STNYNQKFQGRVTITADTSTSTAYMELSSLRSEDTAVYYCTEGYEYDGFDYWGQGT
TVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISKAK
GQPREPQVYTLPPSRDELTKNQVSLTCLVKGFYPSDIAVEWESNGQPENNYKTTPPVL
D  SDGSFFLYSKLTVDKSRWQQGNVFSCSVMHEALHNHYTQKSLSLSPGK (SEQ ID
NO: 43)
 and werein said monoclonal antibody is comprising the following sequence as light chain:

DVVLTQSPLSLPVTLGQPASISCRSSQSIVYSNGNTYLEWYLQRPGQSPRLLIYRVSNR
FSGVPDRFSGSGSGTDFTLKISRVEAEDVGVYYCFQGSHIPYTFGGGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC (SEQ ID No. 44).

Endothelial dysfunction can result from and/or contribute to several disease processes, as occurs in shock, especially septic shock. From preclinical experiments in models of sepsis/ septic shock it is known that administration of the anti-ADM antibody induces an increase of the plasma bio-ADM concentration and that this coincides with an increased survival rate *(*Struck et al. 2013. Intensive Care Med Ex p 1(1):22*).* The mechanism underlying this effect has been described in Geven et al. 2018 *(*Geven et al. 2018. SHOCK 50 (2): 132-140*).* Briefly, the antibody, when administered i.v., due to its size cannot cross the endothelial barrier into the interstitium, but remains in the blood circulation. In contrast, ADM, as a small peptide, can freely diffuse across the endothelial barrier. Thus, the antibody, when administered in a vast molar excess over the endogenous ADM, binds virtually all ADM in the plasma and, as a simple consequence of reaching binding equilibrium, leads to a translocation of ADM from the interstitium to the blood circulation. Interstitially located ADM can bind to vascular smooth muscle cells and induces relaxation resulting in vasodilation. This is reduced by administration of the antibody. On the other hand, ADM in plasma binds to endothelial cells and thereby stabilizes or even restores vascular integrity. Thus, this function is strengthened, when plasma ADM levels increase as a consequence of administration of the antibody, which is a non-neutralizing antibody. Finally, binding of the antibody to ADM reduces the proteolytic decay of ADM. Taken together, the anti-ADM antibody Adrecizumab may restore endothelial function in shock e.g. in septic shock.

If said level of Pro-Adrenomedullin or fragments thereof is above a certain threshold level the anti-ADM antibody or anti-ADM antibody fragment binding to ADM is administered as therapy.

This means in a specific embodiment subject matter of the present invention said anti-ADM antibody or anti-ADM antibody fragment binding to ADM is for therapy, wherein a sample of bodily fluid taken from said patients exhibits an elevated level of proADM and/or fragments thereof having at least 5 amino acids above a certain threshold. Thus, the diagnostic method using said proADM and/or fragments serves as companion diagnostic method.

In a specific embodiment of the invention the threshold is within a threshold range for plasma ADM-NH₂ that is between 50 and 250 pg/ml, preferably between 55 and 200 pg/ml, even more preferred between 60 and 150 pg/ml, even more preferred beween 65 and 100 pg/ml, most preferred a threshold of 70 pg/ml is applied.

In a specific embodiment of the invention the threshold is within a threshold range for plasma MR-proADM that is between 0.5 and 3 nmol/L, preferably between 0.6 and 2 nmol/L, even more preferred between 0.7 and 1 ng/mL, most preferred a threshold of 0.8 nmol/L is applied.

In a specific embodiment of the invention the threshold is within a threshold range for plasma CT-proADM that is between 85 and 500 pmol/L, preferably between 90 and 350 pmol/L, even more preferred between 95 and 250 pmol/L, even more preferred between 100 and 200 pmol/L, most preferred a threshold of 150 pmol/L is applied.

The ADM-NH₂ levels of the present invention or proADM levels or fragments thereof, respectively, have been determined with the described ADM-NH₂ assay, as outlined in the examples (or proADM or fragments thereof assays, respectively) *(*Weber et al. 2017. JALM 2(2):1-4). The DPP3 levels of the present invention have been determined with the described DPP3-assays as outlined in the examples. The mentioned threshold values above might be different in other assays, if these have been calibrated differently from the assay systems used in the present invention. Therefore, the mentioned cut-off values above shall apply for such differently calibrated assays accordingly, taking into account the differences in calibration. One possibility of quantifying the difference in calibration is a method comparison analysis (correlation) of the assay in question with the respective biomarker assay used in the present invention by measuring the respective biomarker (e.g. bio-ADM, DPP3) in samples using both methods. Another possibility is to determine with the assay in question, given this test has sufficient analytical sensitivity, the median biomarker level of a representative normal population, compare results with the median biomarker levels as described in the literature and recalculate the calibration based on the difference obtained by this comparison. With the calibration used in the present invention, samples from normal (healthy) subjects have been measured: median plasma bio-ADM (mature ADM-NH₂) was 24.7 pg/ml, the lowest value 11 pg/ml and the 99^{th} percentile 43 pg/ml *(*Marino et al. 2014. Critical Care 18:R34*).* With the calibration used in the present invention, samples from 5,400 normal (healthy) subjects (swedish single-center prospective population-based Study (MPP-RES)) have been measured: median (interquartile range) plasma DPP3 was 14.5 ng/ml (11.3 ng/ml - 19 ng/ml).

The plasma median MR-proADM concentration in normal (healthy) subjects was 0.41 (interquartile range 0.23 - 0.64) nmol/L *(*Smith et al. 2009. Clin Chem 55:1593-1595*)* using the automated sandwich fluorescence assay for the detection of MR-proADM as described in Caruhel et al. (*C*aruhel et al. 2009. Clin Biochem 42:725-8).

The plasma median concentration of CT-proADM in normal healthy subjects (n=200) was 77.6 pmol/L (min 46.6 pmol/L, max 136.2 pmol/L) and the 95% percentile was 113.8 pmol/L (EP 2 111 552 B1).

In a specific embodiment of the invention a threshold for plasma ADM-NH₂ is the 5fold median concentration, preferably the 4fold median concentration, more preferred the 3fold median concentration, most preferred the 2fold median concentration of a normal healthy population.

In a specific embodiment of the invention a threshold for plasma MR-proADM is the 5fold median concentration, preferably the 4fold median concentration, more preferred the 3fold median concentration, most preferred the 2fold median concentration of a normal healthy population.

In a specific embodiment of the invention a threshold for plasma CT-proADM is the 5fold median concentration, preferably the 4fold median concentration, more preferred the 3fold median concentration, most preferred the 2fold median concentration of a normal healthy population.

In embodiments of the present invention, the anti-ADM antibody or an anti-ADM antibody fragment for use in a treatment of a patient in need thereof, may be administered in a dose of at least 0.5 mg / Kg body weight, particularly at least 1.0 mg/kg body weight, more particularly, from 1.0 to 20.0 mg/kg body weight, e.g., from 2.0 to 10 mg/kg body weight, from 2.0 to 8.0 mg/kg body weight, or from 2.0 to 5.0 mg/kg body weight.

One embodiment of the invention relates to a vasopressor for use in therapy of shock in a subject that either runs into shock or that has developed shock, wherein said subject has a level of DPP3 in a sample of bodily fluid of said subject that is below a predetermined threshold, when determined by any of the methods of predicting or diagnosing a refractory shock according to the present invention.

Another embodiment of the invention relates to a vasopressor for use in therapy of shock in a subject that either runs into shock or that has developed shock, wherein said vasopressor is selected from the group comprising dopamine, norepinephrine, a norepinephrine equivalent, epinephrine, phenylephrine and vasopressin.

Another specific embodiment of the invention relates to a vasopressor for use in therapy of shock in a subject that either runs into shock or that has developed shock, wherein said vasopressor is administered to said subject in a pharmaceutical formulation.

Another embodiment of the invention relates to a vasopressor for use in therapy of shock in a subject that either runs into shock or that has developed shock, wherein said subject has a blood pressure of equal or less than 65 mm Hg.

Another embodiment of the invention relates to an inhibitor of the activity of DPP3 for use in therapy of shock in a subject that either runs into shock or that has developed shock, wherein said subject has a level of DPP3 in a sample of bodily fluid of said subject that is above a predetermined threshold when determined by a method described in the aforementioned embodiments.

Another specific embodiment of the invention relates to an inhibitor of the activity of DPP3 for use in therapy of shock in a subject that either runs into shock or that has developed shock, wherein the inhibitor of the activity of DPP3 is selected from the group comprising anti-DPP3 antibody or anti-DPP3 antibody fragment or anti-DPP3 non-Ig scaffold.

One embodiment of the invention relates to an inhibitor of the activity of DPP3 for use in therapy of shock in a subject that either runs into shock or that has developed shock, wherein said inhibitor has a minimum binding affinity to DPP3 of equal or less than 10⁻⁷ M.

Another embodiment of the invention relates to an inhibitor of the activity of DPP3 for use in therapy of shock in a subject that either runs into shock or that has developed shock, wherein the inhibitor of the activity of DPP3 is an antibody.

Another specific embodiment of the invention relates to an inhibitor of the activity of DPP3 for use in therapy of shock in a subject that either runs into shock or that has developed shock, wherein said inhibitor is an antibody that is monoclonal.

Another embodiment of the invention relates to an inhibitor of the activity of DPP3 for use in therapy of shock in a subject that either runs into shock or that has developed shock, wherein said inhibitor is a monoclonal antibody, wherein the complementarity determining regions (CDR's) in the heavy chain comprises the sequences: SEQ ID NO.: 7, SEQ ID NO.: 8 and/ or SEQ ID NO.: 9 and the complementarity determining regions (CDR's) in the light chain comprises the sequences: SEQ ID NO.: 10, KVS and/or SEQ ID NO.: 11.

Another embodiment of the invention relates to an inhibitor of the activity of DPP3 for use in therapy of shock in a subject that either runs into shock or that has developed shock, wherein said inhibitor is a humanized monoclonal antibody or humanized monoclonal antibody fragment, wherein the heavy chain comprises the sequence: SEQ ID NO.: 12 and wherein the light chain comprises the sequence: SEQ ID NO.: 13.

Said inhibitor of the activity of DPP3 maybe administered by inhalation.

One embodiment of the invention relates to an inhibitor of the activity of DPP3 for use in therapy of shock in a subject that either runs into shock or that has developed shock, wherein said inhibitor is administered in combination with an Angiotensin-Receptor-Agonist and/or precursors thereof.

Another embodiment of the invention relates to an inhibitor of the activity of DPP3 for use in therapy of shock in a subject that either runs into shock or that has developed shock, wherein said Angiotensin-Receptor-Agonist and/or precursors thereof is selected from the group comprising angiotensin I, angiotensin II, angiotensin III, angiotensin IV, in particular angiotensin II.

Another embodiment of the invention relates to an Angiotensin-Receptor-Agonists and/ or precursors thereof and/or inhibitors of the DPP3 activity for use in the treatment of shock in a subject that either runs into shock or that has developed shock, wherein the treatment with said Angiotensin-Receptor-Agonists and/ or precursors thereof and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold.

Another specific embodiment of the invention relates to a vasopressor for use in the treatment of shock in a subject that either runs into shock or that has developed shock, wherein the treatment with said vasopressor is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.

Another specific embodiment of the invention relates to a vasopressor for use in the treatment of shock in a subject that either runs into shock or that has developed shock, wherein the treatment with said vasopressor is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold and wherein the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

Another embodiment of the invention relates to Angiotensin-Receptor-Agonists and/ or precursors thereof and/or inhibitors of the DPP3 activity for use in the treatment of shock in a subject that either runs into shock or that has developed shock, wherein the treatment with said vasopressor is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold, and wherein the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

Another specific embodiment of the invention relates to anti-ADM antibody or anti-ADM antibody fragment for use in the treatment of shock in a subject that either runs into shock or that has developed shock, wherein treatment with said anti-ADM antibody or anti-ADM antibody and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.

In case the level of of Pro-adrenomedullin or fragments thereof in said sample is determined and the level of DPP3 in said sample is determined said determination maybe conducted by a point-of-care device that provides both: a test for determining level of Pro-adrenomedullin or fragments thereof and a test for determining the level of DPP3 in said sample.

Beginning of treatment with an angiotensin-receptor-agonist and/ or a precursor thereof is indicated when a patient has an amount of DPP3 protein and/or DPP3 activity in a sample of bodily fluid that is above a predetermined threshold level.

One embodiment of the invention relates to a method of treatment of shock in a subject that either runs into shock or that has developed shock, the method comprising administering vasopressor to said subject, wherein said subject has a level of DPP3 in a sample of bodily fluid of said subject that is below a predetermined threshold, when determined by a method described in previous embodiments.

Another embodiment of the invention relates to a method of treatment of shock in a subject that either runs into shock or that has developed shock, the method comprising administering vasopressor to said subject, wherein said vasopressor is selected from the group comprising dopamine, norepinephrine, a norepinephrine equivalent, epinephrine, phenylephrine and vasopressin.

A specific embodiment of the invention relates to a method of treatment of shock in a subject that either runs into shock or that has developed shock, the method comprising administering vasopressor to said subject, wherein said vasopressor is administered to said subject in a pharmaceutical formulation.

Another embodiment of the invention relates to a method of treatment of shock in a subject that either runs into shock or that has developed shock, the method comprising administering vasopressor to said subject, wherein said subject has a blood pressure of equal or less than 65 mm Hg.

One embodiment of the invention relates to a method of treatment of shock in a subject that either runs into shock or that has developed shock, the method comprising administering inhibitor of DPP3 activity to said subject, wherein said subject has a level of DPP3 in a sample of bodily fluid of said subject that is above a predetermined threshold when determined by a method described in previous embodiments.

Another embodiment of the invention relates to a method of treatment of shock in a subject that either runs into shock or that has developed shock, the method comprising administering inhibitor of DPP3 activity to said subject, wherein the inhibitor of the activity of DPP3 is selected from the group comprising anti-DPP3 antibody or anti-DPP3 antibody fragment or anti-DPP3 non-Ig scaffold.

Another specific embodiment of the invention relates to a method of treatment of shock in a subject that either runs into shock or that has developed shock, the method comprising administering inhibitor of DPP3 activity to said subject, wherein said inhibitor has a minimum binding affinity to DPP3 of equal or less than 10⁻⁷ M.

Another embodiment of the invention relates to a method of treatment of shock in a subject that either runs into shock or that has developed shock, the method comprising administering inhibitor of DPP3 activity to said subject, wherein the inhibitor of the activity of DPP3 is an antibody.

One embodiment of the invention relates to a method of treatment of shock in a subject that either runs into shock or that has developed shock, the method comprising administering inhibitor of DPP3 activity to said subject, wherein said inhibitor is an antibody that is monoclonal.

One specific embodiment of the invention relates to a method of treatment of shock in a subject that either runs into shock or that has developed shock, the method comprising administering inhibitor of DPP3 activity to said subject, wherein said inhibitor is a monoclonal antibody, wherein the complementarity determining regions (CDR's) in the heavy chain comprises the sequences: SEQ ID NO.: 7, SEQ ID NO.: 8 and/ or SEQ ID NO.: 9 and the complementarity determining regions (CDR's) in the light chain comprises the sequences: SEQ ID NO.: 10, KVS and/or SEQ ID NO.: 11.

Another embodiment of the invention relates to a method of treatment of shock in a subject that either runs into shock or that has developed shock, the method comprising administering inhibitor of DPP3 activity to said subject, wherein said inhibitor is a humanized monoclonal antibody or humanized monoclonal antibody fragment, wherein the heavy chain comprises the sequence: SEQ ID NO.: 12 and wherein the light chain comprises the sequence: SEQ ID NO.: 13.

Another specific embodiment of the invention relates to a method of treatment of shock in a subject that either runs into shock or that has developed shock, the method comprising administering inhibitor of DPP3 activity to said subject, wherein said inhibitor is administered in combination with an Angiotensin-Receptor-Agonist and/or precursors thereof.

Another embodiment of the invention relates to a method of treatment of shock in a subject that either runs into shock or that has developed shock, the method comprising administering inhibitor of DPP3 activity to said subject, wherein said Angiotensin-Receptor-Agonist and/or precursors thereof is selected from the group comprising angiotensin I, angiotensin II, angiotensin III, angiotensin IV, in particular angiotensin II.

Another embodiment of the invention relates to a method of treatment of shock in a subject that either runs into shock or that has developed shock, the method comprising administering Angiotensin-Receptor-Agonists and/or precursors thereof and/or inhibitor of DPP3 activity to said subject, wherein the treatment with said Angiotensin-Receptor-Agonists and/or precursors thereof and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold.

One embodiment of the invention relates to a method of treatment of shock in a subject that either runs into shock or that has developed shock, the method comprising administering vasopressor to said subject, wherein the treatment with said vasopressors is initiated and/or continued when the level of DPP3 in a sample of bodily fluid of said subject is below a certain threshold and/or wherein a treatment with Angiotensin-Receptor-Agonists and/or precursors thereof and/or inhibitors of the DPP3 activity is withheld and/ or terminated if the said determined level of DPP3 is below said predetermined threshold.

One embodiment of the invention relates to a method of treatment of shock in a subject that either runs into shock or that has developed shock, the method comprising administering Angiotensin-Receptor-Agonists and/ or precursors thereof and/or inhibitor of DPP3 activity to said subject, wherein the treatment with said Angiotensin-Receptor-Agonists and/or precursors thereof and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold and wherein the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

One specific embodiment of the invention relates to a method of treatment of shock in a subject that either runs into shock or that has developed shock, the method comprising administering vasopressor to said subject, wherein the treatment with said vasopressors is initiated and/or continued when the level of DPP3 in a sample of bodily fluid of said subject is below a certain threshold and/or wherein a treatment with Angiotensin-Receptor-Agonists and/or precursors thereof and/or inhibitors of the DPP3 activity is withheld and/ or terminated if the said determined level of DPP3 is below said predetermined threshold and wherein the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.

Another embodiment of the invention relates to a method of treatment of shock in a subject that either runs into shock or that has developed shock, the method comprising administering anti-ADM antibody or anti-ADM antibody fragment to said subject, wherein the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold and wherein the treatment with Angiotensin-Receptor-Agonists and/or precursors thereof and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold

Another specific embodiment of the invention relates to a method of treatment of shock in a subject that either runs into shock or that has developed shock, the method comprising administering anti-ADM antibody or anti-ADM antibody fragment to said subject, wherein treatment with said anti-ADM antibody or anti-ADM antibody and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.

An agonist means a chemical that binds to a receptor and activates the receptor to produce a biological response. Receptors can be activated by either endogenous agonists (such as hormones and neurotransmitters) or exogenous agonists (such as drugs), resulting in a biological response. Full agonists bind to and activate a receptor with the maximum response that an agonist can elicit at the receptor. Partial agonists are drugs that bind to and activate a given receptor, but have only partial efficacy at the receptor relative to a full agonist. Potency is the amount of agonist needed to elicit a desired response. The potency of an agonist is inversely related to its EC₅₀ value. The EC₅₀ can be measured for a given agonist by determining the concentration of agonist needed to elicit half of the maximum biological response of the agonist. The EC₅₀ value is useful for comparing the potency of drugs with similar efficacies producing physiologically similar effects. The smaller the EC₅₀ value, the greater the potency of the agonist, the lower the concentration of drug that is required to elicit the maximum biological response.

The treatment with an angiotensin-receptor-agonist and/or a precursor thereof may be continued as long as the patient has an amount of DPP3 protein and/or DPP3 activity in a sample of bodily fluid that is above a predetermined threshold level.

The amount of DPP3 protein and/ or the DPP3 activity is measured at least every 24 hours, preferred every 12 hours, more preferred every 8 hours, even more preferred every 6 hours, even more preferred every 4 hours, even more preferred every 2 hours, even more preferred every hour, most preferred every 30 minutes.

The treatment with an angiotensin-receptor-agonist and/ or a precursor thereof may be discontinued when the patient has an amount of DPP3 protein and/or DPP3 activity in a sample of bodily fluid that is below a predetermined threshold level.

### Angiotensin Therapeutics:

Angiotensin I, also called proangiotensin, is formed by the action of renin on angiotensinogen. Renin cleaves the peptide bond between the leucine (Leu) and valine (Val) residues on angiotensinogen, creating the decapeptide (ten amino acid) (des-Asp) angiotensin I. Angiotensin I is converted to angiotensin II through removal of two C-terminal residues by the enzyme angiotensin-converting enzyme (ACE), primarily through ACE within the lung (but also present in endothelial cells, kidney epithelial cells, and the brain).

Angiotensin II, angiotensin III, and angiotensin IV are peptide hormones naturally produced by the body that regulates blood pressure via vasoconstriction and sodium reabsorption. Hemodynamic effects of angiotensin II administration have been the subject of numerous clinical studies, demonstrating significant effects on systemic and renal blood flow (Harrison-Bernard, L.M., The renal renin-angiotensin system. Adv Physiol Educ, 33(4): 270 (2009*)).*

Angiotensin II is a hormone produced by the renin angiotensin aldosterone system (RAAS) that modulates blood pressure via regulation of vascular smooth muscle tone and extracellular fluid homeostasis. Angiotensin II mediates its effects on the vasculature by inducing vasoconstriction and sodium retention, and so is the target of many therapies for hypertension. In addition to its systemic effects, angiotensin II has a pronounced effect on the efferent arterioles of the kidney, maintaining glomerular filtration when blood flow is decreased. Angiotensin II also regulates sodium reabsorption in the kidney by stimulating Na+/H+ exchangers in the proximal tubule and inducing the release of aldosterone and vasopressin *(*Harrison-Bernard 2009. The renal renin-angiotensin system. Adv Physiol Educ, 33(4):270*).*

The angiotensin II therapeutic that may be used in the compositions and methods of this disclosure may be Asp-Arg-Val-Tyr-Ile-His-Pro-Phe (SEQ ID NO: 13), also called 5-isoleucine angiotensin II. SEQ ID NO: 13 is an octa-peptide naturally present in humans and other species, such as equines, hogs, etc. Isoleucine may be substituted by valine to result in 5-vaiine angiotensin II, Asp-Arg-Val-Tyr-Val-His-Pro-Phe (SEQ ID NO: 14). Other angiotensin II analogues such as [Asn¹-Phe⁴]-angiotensin II (SEQ ID NO: 15), hexapeptide Val-Tyr-Ile-His-Pro-Phe (SEQ ID NO: 16), nonapeptide Asn-Arg-Val-Tyr-Tyr-Val-His-Pro-Phe (SEQ ID NO: 17), [Asn¹ Ile⁵ Ile⁸]-angiotensin II (SEQ ID NO: 18), [Asn¹ -lle⁵- Ala⁸]-angiotensin II (SEQ ID NO: 19), and [Asn¹-diiodoTyr⁴-Ile⁵-angiotensin II (SEQ ID NO: 20) may also be used. Angiotensin II may be synthesized, for example, by solid phase peptide synthesis to incorporate modifications, such as C -terminal amidation. The term "angiotensin II", without further specificity, is intended to refer to any of these various forms, as well as combinations thereof.

In some aspects, a composition comprising angiotensin II may be selected from 5-valine angiotensin II, 5-valine angiotensin II amide, 5-L-isoleucine angiotensin II, and 5-L-isoleucine angiotensin II amide, or a pharmaceutically acceptable salt thereof, preferably manufactured under current good manufacturing conditions (cGMP). In some aspects, the composition may include different forms of angiotensin II in different percentages, e.g., a mixture of hexapeptide and nonapeptide angiotensin II. The composition comprising angiotensin II may be suitable for parenteral administration, e.g., for injection or intravenous infusion.

The sequence of angiotensin II used in the compositions and methods disclosed herein may be homologous to the sequences of angiotensin II described above. In certain aspects, the invention includes isolated, synthetic, or recombinant amino acid sequences that are at least 80%, 85%, 90%, 95%, 97%, 98%, 99% or 100% identical to SEQ ID NO: 13, 14, 15, 16, 17, 18, 19 and/or 20. Any such variant sequences may be used in place of an angiotensin II as described in the preceding paragraph.

Sequence identity for all sequences is determined according to the following method: Percent identity is calculated by multiplying the number of matches in the pair by 100 and dividing by the length of the aligned region, including gaps (Percent Identity = [Matches x 100]/Length of aligned region [with gaps]).

Angiotensin III is a metabolite of angiotensin II with approximately 40% of the activity of angiotensin II. An angiotensin III therapeutic that may be used for in the compositions and methods of this disclosure may be Arg-Val-Tyr-Ile-His-Pro-Phe (SEQ ID NO: 21). SEQ ID NO: 21 is an hepta-peptide naturally present in humans and other species, such as equines, hogs, etc. Isoleucine may be substituted by valine to result in Arg-Val-Tyr-Val-His-Pro-Phe (SEQ ID NO: 22). Other angiotensin III analogues such as [Phe³]-angiotensin III (SEQ ID NO: 23), [Ile⁴-Ala⁷]-angiotensin III (SEQ ID NO: 24), and [diiodoTyr³-Ile⁴]-angiotensin III (SEQ ID NO: 25) may also be used. Angiotensin III may be synthesized, for example, by solid phase peptide synthesis to incorporate modifications, such as C-terminal amidation. The term "angiotensin III", without further specificity, is intended to refer to any of these various forms, as well as combinations thereof.

In some aspects, a composition comprising angiotensin III may be selected from 4-valine angiotensin III, 4-valine angiotensin III amide, 4-L-isoleucine angiotensin III, and 4-L-isoleucine angiotensin III amide, or a pharmaceutically acceptable salt thereof, preferably manufactured under current good manufacturing conditions (cGMP). A composition comprising angiotensin III may be suitable for parenteral administration, e.g., for injection or intravenous infusion.

The sequence of angiotensin III used in the compositions and methods disclosed herein may be homologous to the sequences of angiotensin III described above. In certain aspects, the invention includes isolated, synthetic, or recombinant amino acid sequences that are at least 80%, 85%, 90%, 95%, 97%, 98%, 99% or 100% identical to SEQ ID NO: 21, 22, 23, 24 and/or 25. Any such variant sequences may be used in place of an angiotensin II as described in the preceding paragraph.

Angiotensin IV is a metabolite of angiotensin III with less activity than angiotensin II. An angiotensin IV therapeutic that may be used for in the compositions and methods of this disclosure may be Val-Tyr-Ile-His-Pro-Phe (SEQ ID NO: 26). SEQ ID NO: 26 is a hexapeptide naturally present in humans and other species. Isoleucine may be substituted by valine to result in Val-Tyr-Val-His-Pro-Phe (SEQ ID NO: 27). Other angiotensin IV analogues such as [Phe²]-angiotensin III (SEQ ID NO: 28), [Ile³-AIa⁶]-angiotensin IV (SEQ ID NO: 29), and [diiodoTyr²-Ile³]-angiotensin IV (SEQ ID NO: 30) may also be used. Angiotensin IV may be synthesized, for example, by solid phase peptide synthesis to incorporate modifications, such as C-terminal amidation. The term "angiotensin IV", without further specificity, is intended to refer to any of these various forms, as well as combinations thereof.

In some aspects, a composition comprising angiotensin IV may be selected from 3-valine angiotensin IV, 3-valine angiotensin IV amide, 3-L-isoleucine angiotensin IV, and 3-L-isoieucine angiotensin IV amide, or a pharmaceutically acceptable salt thereof, preferably manufactured under current good manufacturing conditions (cGMP). A composition comprising angiotensin IV may be suitable for parenteral administration, e.g., for injection or intravenous infusion.

The sequence of angiotensin IV used in the compositions and methods disclosed herein may be homologous to the sequences of angiotensin IV described above. In certain aspects, the invention includes isolated, synthetic, or recombinant amino acid sequences that are at least 80%, 85%, 90%, 95%, 97%, 98%, 99% or 100% identical to SEQ ID NO: 25, 26, 27, 28, 29 and/or 30. Any such variant sequences may be used in place of an angiotensin IV as described in the preceding paragraph.

An angiotensin II, angiotensin III, or angiotensin IV therapeutic may be used as any suitable salt (e.g. acetate), deprotected form, acetylated form, deacetylated form, and/or prodrug form of the above-mentioned peptides, including pegylated forms of the peptides or conjugates as disclosed in US Patent Publication 2011/0081371 (incorporated by reference). The term "prodrug" refers to any precursor compound which is able to generate or to release the above-mentioned peptide under physiological conditions. Such prodrugs or precursors may be larger peptides which are selectively cleaved in order to form the peptide of the invention. For example, in some aspects, the prodrug or precursor may be angiotensinogen, angiotensin I, or its homologues that may result in angiotensin II by the action of certain endogenous or exogenous enzymes. Further prodrugs or precursors include peptides with protected amino acids, e.g., having protecting groups at one or more carboxylic acid and/or amino groups. Suitable protecting groups for amino groups include the benzyloxycarbonyl, t-butyloxycarbonyl (BOC), fluorenylmethyloxycarbonyl (FMOC), formyl, and acetyl or acyl group. Suitable protecting groups for the carboxylic acid group include esters such as benzyl esters or t-butyl esters. The present invention also contemplates the use of angiotensin II, angiotensin III, angiotensin IV and/or precursor peptides having amino acid substitutions, deletions, additions, the substitutions and additions including the standard D and L amino acids and modified amino acids, such as, for example, amidated and acetylated amino acids, wherein the therapeutic activity of the base peptide sequence is maintained at a pharmacologically useful level.

In a preferred embodiment the angiotensin II is angiotensin II acetate. Angiotensin II acetate is L-Aspartyl-L-arginyl-L-valyl-Ltyrosyl-L-isoleucyl-L-histidyl-L-prolyl-L-phenylalanine, acetate salt. The counter ion acetate is present in a non-stoichiometric ratio. The molecular formula of angiotensin II acetate is C₅₀H₇₁N₁₃O₁₂ • (C₂H₄O₂)n; (n=number of acetate molecules; theoretical n = 3) with an average molecular weight of 1046.2 (as free base).

One embodiment of the invention is an angiotensin-receptor-agonist and/ or a precursor thereof for use in the treatment of a disease in a subject, wherein said subject has an amount of DPP3 protein and/or DPP3 activity in a sample of bodily fluid that is above a predetermined threshold, wherein the determination of the amount of DPP3 protein and/or DPP3 activity in a sample of bodily fluid is used as therapy guidance and/or therapy monitoring for said treatment with Angiotensin-Receptor-Agonist and/ or a precursor thereof.

In one embodiment of the invention the amount of DPP3 protein and/or DPP3 activity in a sample of bodily fluid is determined before treatment of the subject with an angiotensin-receptor-agonist and/ or a precursor thereof. In one embodiment of the invention the amount of DPP3 protein and/or DPP3 activity in a sample of bodily fluid is determined during treatment of the subject with an angiotensin-receptor-agonist and/ or a precursor thereof at least once during treatment, preferably at least twice or preferably at least once daily during treatment. In one embodiment of the invention the amount of DPP3 protein and/or DPP3 activity in a sample of bodily fluid is determined after treatment of the subject with an angiotensin-receptor-agonist and/ or a precursor thereof. In one embodiment of the invention the amount of DPP3 protein and/or DPP3 activity in a sample of bodily fluid is determined before and/or during and/or after treatment of the subject with an angiotensin-receptor-agonist and/ or a precursor thereof.

One embodiment of the invention is an angiotensin-receptor-agonist and/ or a precursor thereof for use in the treatment ofshock in a subject that either runs into shock or that has developed shock , wherein said subject has an amount of DPP3 protein and/or DPP3 activity in a sample of bodily fluid that is above a predetermined threshold, wherein said sample of bodily fluid of said subject is selected from whole blood, blood plasma and blood serum.

One embodiment of the invention is an angiotensin-receptor-agonist and/ or a precursor thereof for use in the treatment of shock in a subject that either runs into shock or that has developed shock, wherein said Angiotensin-Receptor-Agonist and/ or a precursor thereof is administered to said subject in a pharmaceutical formulation.

The term "pharmaceutical formulation" means a pharmaceutical (active) ingredient in combination with at least one pharmaceutically acceptable excipient.

One embodiment of the invention is an angiotensin-receptor-agonist and/ or a precursor thereof for use in the treatment of shock in a subject that either runs into shock or that has developed shock , wherein said Angiotensin-Receptor-Agonist and/ or a precursor thereof is Angiotensin II and said pharmaceutical formulation is a solution, preferably a ready-to-use solution. In another embodiment subject of the present invention is further a pharmaceutical formulation according to the present invention wherein said pharmaceutical formulation is in a dried state to be reconstituted before use.

The pharmaceutical formulation as disclosed herein may also contain diluents, fillers, salts, buffers, stabilizers, solubilizers, and other materials well known in the art. The term "pharmaceutically acceptable carrier" refers to a non-toxic carrier that may be administered to a patient, together with a therapeutically effective substance (such as angiotensin II) of this invention, and which does not destroy the pharmacological activity of the therapeutically effective substance. The term "pharmaceutically acceptable" means a non-toxic material that does not interfere with the effectiveness of the biological activity of the active ingredient(s).

The characteristics of the carrier will depend on the route of administration. The term "excipient" refers to an additive in a formulation or composition that is not a pharmaceutically active ingredient.

The term "pharmaceutical (active) ingredient" means a therapeutic composition which can be optionally combined with pharmaceutically acceptable excipients to provide a pharmaceutical formulation or dosage form.

One of skill in the art would appreciate that the choice of any one excipient may influence the choice of any other excipient. For example, the choice of a particular excipient may preclude the use of one or more additional excipients because the combination of excipients would produce undesirable effects. Excipients of the invention may include, but are not limited to, co-solvents, solubilizing agents, buffers, pH adjusting agents, bulking agents, surfactants, encapsulating agents, tonicity-adjusting agents, stabilizing agents, protectants, and viscosity modifiers.

In some aspects, it may be beneficial to include a pharmaceutically acceptable carrier in the compositions as dislosed herein.

In some aspects, it may be beneficial to include a solubilizing agent in the compositions of the invention. Solubilizing agents may be useful for increasing the solubility of any of the components of the formulation or composition, including a therapeutically effective substance (e.g., angiotensin **II,** angiotensin **III,** or angiotensin IV) or an excipient. The solubilizing agents described herein are not intended to constitute an exhaustive list, but are provided merely as exemplary solubilizing agents that may be used in the compositions of the invention. In certain aspects, solubilizing agents include, but are not limited to, ethyl alcohol, tert-butyl alcohol, polyethylene glycol, glycerol, methylparaben, propylparaben, polyethylene glycol, polyvinyl pyrrolidone, and any pharmaceutically acceptable salts and/or combinations thereof.

In some aspects, it may be beneficial to adjust the pH of the compositions by including a pH-adjusting agent in the compositions of the invention. Modifying the pH of a formulation or composition may have beneficial effects on, for example, the stability or solubility of a therapeutically effective substance, or may be useful in making a formulation or composition suitable for parenteral administration. pH-adjusting agents are well known in the art. Accordingly, the pH-adjusting agents described herein are not intended to constitute an exhaustive list, but are provided merely as exemplary pH-adjusting agents that may be used in the compositions of the invention. pH-adjusting agents may include, for example, acids and bases. In some aspects, a pH-adjusting agent includes, but is not limited to, acetic acid, hydrochloric acid, phosphoric acid, sodium hydroxide, sodium carbonate, and combinations thereof.

The pH of the compositions as disclosed herein may be any pH that provides desirable properties for the formulation or composition. Desirable properties may include, for example, therapeutically effective substance (e.g., angiotensin II, angiotensin III, or angiotensin IV) stability, increased therapeutically effective substance retention as compared to compositions at other pHs, and improved filtration efficiency. In some aspects, the pH of the compositions of the invention may be from about 3.0 to about 9.0, e.g., from about 5.0 to about 7.0. In particular aspects, the pH of the compositions of the invention may be 5.5±0.1, 5.6±0.1, 5.7±0.1, 5.8±0.1, 5.9±0.1, 6.0±0.1, 6.1±0.1, 6.2±0.1, 6.3±0.1, 6.4±0.1, or 6.5±0.1.

In some aspects, it may be beneficial to buffer the pH by including one or more buffers in the compositions. In certain aspects, a buffer may have a pKa of, for example, about 5.5, about 6.0, or about 6.5. One of skill in the art would appreciate that an appropriate buffer may be chosen for inclusion in compositions of the invention based on its pKa and other properties.

Buffers are well known in the art. Accordingly, the buffers described herein are not intended to constitute an exhaustive list, but are provided merely as exemplary buffers that may be used in the compositions of the invention. In certain aspects, a buffer may include one or more of the following: Tris, Tris HC1, potassium phosphate, sodium phosphate, sodium citrate, sodium ascorbate, combinations of sodium and potassium phosphate, Tris/Tris HC1, sodium bicarbonate, arginine phosphate, arginine hydrochloride, histidine hydrochloride, cacodylate, succinate, 2-(N-morpholino)ethanesulfonic acid (MES), maleate, bis-tris, phosphate, carbonate, and any pharmaceutically acceptable salts and/or combinations thereof.

**In** some aspects, it may be beneficial to include a surfactant in the compositions of the invention. Surfactants, in general, decrease the surface tension of a liquid composition. This may provide beneficial properties such as improved ease of filtration. Surfactants also may act as emulsifying agents and/or solubilizing agents. Surfactants are well known in the art. Accordingly, the surfactants described herein are not intended to constitute an exhaustive list, but are provided merely as exemplary surfactants that may be used in the compositions of the invention. Surfactants that may be included include, but are not limited to, sorbitan esters such as polysorbates (e.g., polysorbate 20 and polysorbate 80), lipopolysaccharides, polyethylene glycols (e.g., PEG 400 and PEG 3000), poloxamers (i.e., pluronics), ethylene oxides and polyethylene oxides (e.g., Triton X-100), saponins, phospholipids (e.g., lecithin), and combinations thereof.

**In** some aspects, it may be beneficial to include a tonicity-adjusting agent in the compositions of the invention. The tonicity of a liquid composition is an important consideration when administering the composition to a patient, for example, by parenteral administration. Tonicity-adjusting agents, thus, may be used to help make a formulation or composition suitable for administration. Tonicity-adjusting agents are well known in the art. Accordingly, the tonicity-adjusting agents described herein are not intended to constitute an exhaustive list, but are provided merely as exemplary tonicity-adjusting agents that may be used in the compositions of the invention. Tonicity-adjusting agents may be ionic or non-ionic and include, but are not limited to, inorganic salts, amino acids, carbohydrates, sugars, sugar alcohols, and carbohydrates. Exemplary inorganic salts may include sodium chloride, potassium chloride, sodium sulfate, and potassium sulfate. An exemplary amino acid is glycine. Exemplary sugars may include sugar alcohols such as glycerol, propylene glycol, glucose, sucrose, lactose, and mannitol.

**In** some aspects, it may be beneficial to include a stabilizing agent in the compositions of the invention. Stabilizing agents help increase the stability of a therapeutically effective substance in compositions of the invention. This may occur by, for example, reducing degradation or preventing aggregation of a therapeutically effective substance. Without wishing to be bound by theory, mechanisms for enhancing stability may include sequestration of the therapeutically effective substance from a solvent or inhibiting free radical oxidation of the anthracycline compound. Stabilizing agents are well known in the art. Accordingly, the stabilizing agents described herein are not intended to constitute an exhaustive list, but are provided merely as exemplary stabilizing agents that may be used in the compositions of the invention. Stabilizing agents may include, but are not limited to, emulsifiers and surfactants.

The compositions as disclosed herein can be administered in a variety of conventional ways. In some aspects, the compositions of the invention are suitable for parenteral administration. These compositions may be administered, for example, intraperitoneally, intravenously, intrarenally, intrathecally or by inhalation. In some aspects, the compositions of the invention are injected intravenously. One of skill in the art would appreciate that a method of administering a therapeutically effective substance formulation or composition of the invention would depend on factors such as the age, weight, and physical condition of the patient being treated, and the disease or condition being treated. The skilled worker would, thus, be able to select a method of administration optimal for a patient on a case-by-case basis.

Angiotensin-receptor-agonist and/ or a precursor thereof can be administered in any suitable way, but are typically administered by continuous infusion. Accordingly, increasing or decreasing a rate of administration can be accomplished by changing the rate of flow of an intravenous drip, changing the concentration of the agent in an intravenous drip, etc. However, the manner in which the rate of administration is changed will depend on the mode of administration of the therapeutic. Where the therapeutic is administered transmucosally or transdermally, the rate may be increased by changing to a higher-release-rate patch or transdermal composition for example. Where the therapeutic is administered orally, the rate may be increased by switching to a higher-dose form, administering additional doses, or administering controlled-release dosage forms with a higher rate of release, for example.

Where the therapeutic is administered by inhalation, the rate may be increased by administering additional boluses, a more concentrated bolus, or a faster-release bolus, for example. Other modes of administration (via subcutaneous injection pump, suppository, etc.) can be modulated in analogous fashions, and decreasing the rate of administration can be accomplished by doing the opposite of an action that would increase the rate of administration of the therapeutic.

One embodiment of the invention is an angiotensin-receptor-agonist and/ or a precursor thereof for use in the treatment of shock in a subject that either runs into shock or that has developed shock, wherein said Angiotensin-Receptor-Agonist and/ or a precursor thereof is Angiotensin II that is administered at a rate between 0.1 and 200 ng/kg/min, preferably between 1 and 100 ng/kg/min, more preferred between 2 and 80 ng/kg/min, even more preferred between 5 and 60 ng/kg/min, even more preferred between 10 and 50 ng/kg/min, even more preferred between 15 and 40 ng/kg/min most preferred at a rate of 20 ng/kg/min.

In a specific embodiment of the present disclosure the starting dosage (initial rate) of angiotensin II is 80 ng/kg/min, more preferred 40 ng/kg/min, most preferred 20 ng/kg/min via continuous intravenous infusion.

In another specific embodiment of the invention for titration of angiotensin II the blood pressure response (e.g. mean arterial pressure; MAP) is monitored. Titration of angiotensin II may be carried out every 60 minutes, more preferred every 45 minutes, even more preferred every 30 minutes, even more preferred every 15 minutes, even more preferred every 10 minutes, most preferred every 5 minutes. Angiotensin II titration may be carried out by increments of up to 40 ng/kg/min, more preferred up to 20 ng/kg/min, most preferred up to 15 ng/kg/min as needed to achieve or maintain target blood pressure. In another preferred embodiment the dosage should not exceed 80 ng/kg/min of angiotensin II during the first 3 hours of treatment. It is most preferred that maintenance doses should not exceed 40 ng/kg/min andd oses as low as 1.25 ng/kg/min may be used.

In some embodiments, the patient has an initial mean arterial pressure (MAP) of no more than about 40 mm Hg, about 45 mm Hg, about 50 mm Hg, 55 mm Hg, about 60 mm Hg, about 65 mm Hg, about 70 mm Hg, or about 75 mm Hg prior to administering the composition. The method may comprise measuring a mean atrial blood pressure of the patient and increasing the rate of administering angiotensin II if the mean arterial blood pressure is less than about 40 mm Hg, about 45 mm Hg, about 50 mm Hg, 55 mm Hg, about 60 mm Hg, about 65 mm Hg, about 70 mm Hg, or about 75 mm Hg.

In one embodiment, the patient may be receiving a vasopressor (e.g., a catecholamine, such as norepinephrine, a norepinephrine equivalent, epinephrine, dopamine, phenylephrine) or a combination thereof. In some embodiments, the vasopressor is vasopressin (e.g., terlipressin, argipressin, desmopressin, felypressin, lypressin, or ornipressin).

One embodiment of the invention is an angiotensin-receptor-agonist and/ or a precursor thereof for use in the treatment of shock in a subject that either runs into shock or that has developed shock, wherein said angiotensin-receptor-agonist and/ or a precursor thereof, in particular Angiotensin II, is administered in combination with an inhibitor of DPP3.

One embodiment of the invention is an angiotensin-receptor-agonist and/ or a precursor thereof for use in the treatment of shock in a subject that either runs into shock or that has developed shock, in combination with an inhibitor of DPP3, wherein said inhibitor of DPP3 is selected from the group comprising an anti-DPP3 antibody or anti-DPP3 antibody fragment or anti-DPP3 non-Ig scaffold.

In accordance with the invention the "anti-DPP3 antibody" is an antibody that binds specifically to DPP3, an "anti-DPP3 antibody fragment" is a fragment of said anti-DPP3 antibody, wherein said fragment binds specifically to DPP3. An "anti-DPP3 non-Ig scaffold" is a non-Ig scaffold that binds specifically to DPP3.

One embodiment of the invention is an angiotensin-receptor-agonist and/ or a precursor thereof for use in the treatment of shock in a subject that either runs into shock or that has developed shock, in combination with an inhibitor of DPP3, wherein said inhibitor of DPP3 is an anti-DPP3 antibody or anti-DPP3 antibody fragment or anti-DPP3 non-Ig scaffold that binds to SEQ ID NO. 1, in particular that binds to SEQ ID NO. 2.

One embodiment of the invention is an angiotensin-receptor-agonist and/ or a precursor thereof for use in the treatment of shock in a subject that either runs into shock or that has developed shock, , in combination with an inhibitor of DPP3, wherein said inhibitor of DPP3 is an antibody or fragment or scaffold that exhibits a minimum binding affinity to DPP3 of equal or less than 10⁻⁷ M.

In accordance with the present invention, the person skilled in the art is well aware that the binding affinity of the herein disclosed DPP3 binder to DPP3 may be measured by various suitable assays known in the art. Respective examples are given below, but these shall be not construed as limiting possibilities to measure binding affinity of the herein disclosed DPP3 binder to DPP3.

For example, the binding affinity of the DPP3 binder to an epitope may be determined. A binding assay may be performed to detect and/or quantitate antibody binding to e.g. the immunization peptide of the respective binder. For example, this immunization peptide may be immobilized upon a solid phase. The test sample (e.g. antibody solution) is passed over the immobile immunization peptide, and bound antibody can be detected. For the purposes of the present description, the term "solid phase" may be used to include any material or vessel in which or on which the assay may be performed and includes, but is not limited to, porous materials, nonporous materials, test tubes, wells, slides, magnetic beads etc.

Exemplary detection methods:
- Label antibody before contacting with solid phase and detect respective label (fluorescence, chemiluminescence, enzymatic etc.).
- Use labeled secondary antibody against specific Fc part of sample-antibody. Incubate solid phase bound antibody with secondary antibody (e.g. anti human IgG, anti murine IgG) and detect respective label (fluorescence, chemiluminescence, enzymatic etc.).
- Use a labeled antibody as competitor for solid phase binding (e.g. labeled AK1967).
- Quantify binding affinity by decrease of signal.

Another method to determine the affinity of antibodies to DPP3, the kinetics of binding of DPP3 to immobilized antibody may be determined by means of label-free surface plasmon resonance using a Biacore 2000 system (GE Healthcare Europe GmbH, Freiburg, Germany). Reversible immobilization of the antibodies may be performed using an anti-mouse Fc antibody covalently coupled in high density to a CM5 sensor surface (mouse antibody capture kit; GE Healthcare) *(*Lorenz et al. 2011. Antimicrob Agents Chemother. 55(1): 165-173*).*

One embodiment of the invention is an angiotensin-receptor-agonist and/ or a precursor thereof for use in the treatment of shock in a subject that either runs into shock or that has developed shock, in combination with an inhibitor of DPP3, wherein said inhibitor of DPP3 is an antibody or fragment or scaffold that is monospecific, in one embodiment said inhibitor of DPP3 is an antibody or fragment or scaffold that is monoclonal.

Monospecific antibodies or fragments or non-Ig scaffolds according to the invention are antibodies or fragments or non-Ig scaffolds that all have affinity for the same antigen. Monoclonal antibodies are monospecific, but monospecific antibodies may also be produced by other means than producing them from a common germ cell.

In a specific embodiment said capture-binder that binds to full-length DPP3 specifically inhibits less than 50% DPP3 activity in a liquid phase assay, preferably less than 40%, more preferably 30 %. For definition of liquid phase assay see above. In one specific embodiment to prevent inhibition of DPP3 the capture-binder should not bind DPP3 in the area around the active center and substrate binding region (amino acids 316 - 669 of SEQ ID No. 1).

One embodiment of the invention is an angiotensin-receptor-agonist and/ or a precursor thereof for use in the treatment of shock in a subject that either runs into shock or that has developed shock, in combination with an inhibitor of DPP3, wherein said inhibitor of DPP3 is an antibody or fragment or scaffold that binds to full-length DPP3 and inhibits activity of DPP3 of at least 10%, or at least 50%, more preferred at least 60%, even more preferred more than 70 %, even more preferred more than 80 %, even more preferred more than 90 %, even more preferred more than 95 %.

Inhibition of DPP3 activity in a liquid phase assay by a binder may be determined as followed: Possible DPP3 capture-binders are incubated with recombinant or purified native DPP3 and specific DPP3 substrates in a liquid phase assay. Preferably, as capture-binder for the ECA is chosen the one with the least inhibitory ability. The capture-binder should inhibit DPP3 activity less than 50 %, preferably less than 40 %, preferably less than 30 %. The specific liquid phase DPP3 activity assay to determine the inhibitory ability of possible capture-binders comprises the following steps:
- Incubation of 25 ng/ ml native human DPP3 with 5 µg/ ml of the respective capture-binder and buffer control in 50 mM Tris-HCl, pH 7,5 and 100 µM ZnCl₂ for 1 hour at room temperature.
- Addition of the fluorogenic substrate Arg-Arg-βNA (20 µl, 2 mM).
- Incubation at 37 °C and monitoring the generation of free βNA in a Twinkle LB 970 microplate fluorometer (Berthold Technologies GmbH) over 1 hour. Fluorescence of βNA is detected by exciting at 340 nm and measuring emission at 410 nm.
- Slopes (in RFU/ min) of increasing fluorescence of the different samples are calculated. The slope of native human DPP3 with buffer control is appointed as 100 % activity. The inhibitory ability of a possible capture-binder is defined as the decrease of native human DPP3 activity by incubation with said capture-binder in percent.

In a liquid phase assay samples of bodily fluids are directly subjected to fluorogenic substrates (e.g. Arg-Arg-β-NA). Since there are many different amino peptidases in the plasma *(Sanderink et al. 1988*), it is possible that the used substrate is cleaved by peptidases other than DPP3. To circumvent this problem one preferred method of detecting specific DPP3 activity is the use of an enzyme capture activity assay.

In one specific embodiment determination of active DPP3 in an enzyme capture assay comprises the steps:
- Contacting said sample with a capture-binder that binds to full-length DPP3 but preferably inhibits DPP3 activity in a liquid phase assay less than 50%, preferably less than 40%, more preferably 30 %. To prevent inhibition of DPP3 the capture-binder should not bind DPP3 in the area around the active center and substrate binding region (amino acids 316 - 669 of SEQ ID No. 1),
- separating DPP3 bound to said capture binder from bodily fluid sample,
- adding substrate of DPP3 to said separated DPP3,
- quantifying DPP3 activity by measuring the conversion of the substrate of DPP3.

An "antibody" according to the present invention is a protein including one or more polypeptides substantially encoded by immunoglobulin genes that specifically binds an antigen. The recognized immunoglobulin genes include the kappa, lambda, alpha (IgA), gamma (IgG₁, IgG₂, IgG₃, IgG₄), delta (IgD), epsilon (IgE) and mu (IgM) constant region genes, as well as the myriad immunoglobulin variable region genes. Full-length immunoglobulin light chains are generally about 25 kDa or 214 amino acids in length.

Full-length immunoglobulin heavy chains are generally about 50 kDa or 446 amino acids in length. Light chains are encoded by a variable region gene at the NH₂-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and F(ab')₂, as well as bifunctional hybrid antibodies and single chains *(e.g.,* Lanzavecchia et al., Eur. J. Immunol. 17:105,1987*;* Huston et al., Proc. Natl. Acad. Sci. U.S.A., 85:5879-5883, 1988*;* Bird et al., Science 242:423-426, 1988*;* Hood et al., Immunology, Benjamin, N.Y., 2nd ed., 1984*;* Hunkapiller and Hood, Nature 323:15-16,1986*).*

An immunoglobulin light or heavy chain variable region includes a framework region interrupted by three hypervariable regions, also called complementarity determining regions (CDR's) (see, Sequences of Proteins of Immunological Interest, E. Kabat et al., U.S. Department of Health and Human Services, 1983). As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

"Chimeric antibodies" are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, *e.g.,* see U.S. Patent No. 5,807,715. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor."

In one embodiment of the invention, all the CDRs are from the donor immunoglobulin in a humanized immunoglobulin. Constant regions need not be present, but if they are, they must be substantially identical to human immunoglobulin constant regions, *i.e.,* at least about 85-90%, such as about 95% or more identical. Hence, all parts of a humanized immunoglobulin, except possibly the CDRs, are substantially identical to corresponding parts of natural human immunoglobulin sequences.

A "humanized antibody" in accordance with the invention is an antibody comprising a humanized light chain and a humanized heavy chain immunoglobulin. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (e.g., see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.,* Dower et al., PCT Publication No. WO 91/17271; McCafferty et al., PCT Publication No. WO 92/001047; and Winter, PCT Publication No. WO 92/20791), or selected from a human combinatorial monoclonal antibody library (see the Morphosys website). Human antibodies can also be prepared by using transgenic animals carrying a human immunoglobulin gene (for example, see Lonberg et al., PCT Publication No. WO 93/12227; and Kucherlapati, PCT Publication No. WO 91/10741).

Thus, the anti-DPP3 antibody or anti-DPP3 antibody fragment in accordance with the invention may have the formats known in the art. Examples are human antibodies, monoclonal antibodies, humanized antibodies, chimeric antibodies, CDR-grafted antibodies or antibody fragments thereof, but not limited to.

In a specific embodiment of the invention the anti-DPP3 antibody is a monoclonal antibody or a fragment thereof. In one embodiment of the invention the anti-DPP3 antibody or the anti-DPP3 antibody fragment is a human or humanized antibody or derived therefrom. In one specific embodiment one or more (murine) CDR's are grafted into a human antibody or antibody fragment.

In a preferred embodiment antibodies according to the present invention are recombinantly produced antibodies as *e.g.* IgG, a typical full-length immunoglobulin, or antibody fragments containing at least the F-variable domain of heavy and/or light chain as e.g. chemically coupled antibodies (fragment antigen binding) including but not limited to Fab-fragments including Fab minibodies, single chain Fab antibody, monovalent Fab antibody with epitope tags, e.g. Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains, *e.g.* Fab-dHLX-FSx2; F(ab')2-fragments, scFv-fragments, multimerized multivalent and/or multispecific scFv-fragments, bivalent and/or bispecific diabodies, BITE^{®} (bispecific T-cell engager), trifunctional antibodies, polyvalent antibodies, *e.g.* from a different class than G; single-domain antibodies, *e.g.* nanobodies derived from camelid or fish immunoglobulines and numerous others.

In addition to anti-DPP3 antibodies or anti-DPP3 antibody fragments, other biopolymer scaffolds, so called non-Ig scaffolds, are well known in the art to complex a target molecule and have been used for the generation of highly target specific biopolymers. Examples are aptamers, spiegelmers, anticalins and conotoxins.

Non-Ig scaffolds with the context of the invention may be protein scaffolds and may be used as antibody mimics as they are capable to bind to ligands or antigens. Non-Ig scaffolds may be selected from the group comprising tetranectin-based non-Ig scaffolds (e.g. described in US 2010/0028995), fibronectin scaffolds (e.g. described in EP 1266 025; lipocalin-based scaffolds *(e.g.* described in WO 2011/154420); ubiquitin scaffolds *(e.g.* described in WO 2011/073214), transferring scaffolds (e.g. described in US 2004/0023334), protein A scaffolds *(e.g.* described in EP 2231860), ankyrin repeat based scaffolds *(e.g.* described in WO 2010/060748), microprotein (preferably microproteins forming a cystine knot) scaffolds *(e.g.* described in EP 2314308), Fyn SH3 domain based scaffolds *(e.g.* described in WO 2011/023685), EGFR-A-domain based scaffolds (e.g. described in WO 2005/040229) and Kunitz domain based scaffolds (e.g. described in EP 1941867). Non-Ig scaffolds may be peptide or oligonucleotide aptamers. Aptamers are usually created by selecting them from a large random sequence pool and are either short strands of oligonucleotides (DNA, RNA or XNA; *Xu et al. 2010, Deng et al. 2014)* or short variable peptide domains attached to a protein scaffold *(Li et al. 2011).*

In an alternative embodiment the anti-DPP3 antibody format is selected from the group comprising Fv fragment, scFv fragment, Fab fragment, scFab fragment, F(ab)₂ fragment and scFv-Fc Fusion protein. In another preferred embodiment the antibody format is selected from the group comprising scFab fragment, Fab fragment, scFv fragment and bioavailability optimized conjugates thereof, such as PEGylated fragments.

With the context of the invention, the term "antibody" generally comprises monoclonal and polyclonal antibodies and binding fragments thereof, in particular Fc-fragments as well as so called "single-chain-antibodies" *(Bird et al. 1988),* chimeric, humanized, in particular CDR-grafted antibodies, and di- or tetrabodies *(Holliger et al. 1993).* Also comprised are immunoglobulin-like proteins that are selected through techniques including, for example, phage display to specifically bind to the molecule of interest contained in a sample. In this context the term "specific binding" refers to antibodies raised against the molecule of interest or a fragment thereof. An antibody is considered to be specific, if its affinity towards the molecule of interest or the aforementioned fragment thereof is at least preferably 50-fold higher, more preferably 100-fold higher, most preferably at least 1000-fold higher than towards other molecules comprised in a sample containing the molecule of interest. It is well known in the art how to make antibodies and to select antibodies with a given specificity.

In a specific embodiment of the invention said anti-DPP3 antibody or anti-DPP3 antibody fragment binding to an epitope according to SEQ ID NO.: 2, wherein said epitope is comprised in a DPP3 protein or functional derivative thereof is a monoclonal antibody or a monoclonal antibody fragment thereof. In one embodiment of the invention the anti-DPP3 antibody or the anti-DPP3 antibody fragment binding to an epitope according to SEQ ID NO.: 2, wherein said epitope is comprised in a DPP3 protein or functional derivative thereof is a human or humanized antibody or derived therefrom or humanized antibody fragment or derived therefrom.

In one specific embodiment one or more (murine) CDR's are grafted into a human antibody or antibody fragment.

In another aspect of the invention, the provided subject matter is a human CDR-grafted anti-DPP3 antibody or anti-DPP3 antibody fragment thereof that is directed to and binding to an epitope according to SEQ ID NO.: 2, wherein said epitope is comprised in a DPP3 protein or a functional derivative thereof, and wherein said human CDR-grafted anti-DPP3 antibody or anti-DPP3 antibody fragment thereof comprises an antibody heavy chain variable region (H chain) comprising:
SEQ ID NO.: 4
and/or further comprises an antibody light chain variable region (L chain) comprising:
   SEQ ID NO.: 5.

Further subject matter of the present invention in another aspect is a human CDR-grafted anti-DPP3 antibody or anti-DPP3 antibody fragment thereof that is directed to and binding to an epitope according to SEQ ID NO.: 2, wherein said epitope is comprised in a DPP3 protein or a functional derivative thereof, and wherein the said human CDR-grafted anti-DPP3 antibody or anti-DPP3 antibody fragment thereof comprises an antibody heavy chain variable region (H chain) comprising:
SEQ ID NO.: 11
and/or further comprises an antibody light chain variable region (L chain) comprising:
   SEQ ID NO.: 12.

In one specific embodiment of the invention subject matter of the present invention is a human monoclonal anti-DPP3 antibody or monoclonal anti-DPP3 antibody fragment thereof that is directed to and binding to an epitope according to SEQ ID NO.: 2, wherein said epitope is comprised in a DPP3 protein or a functional derivative thereof, and wherein the heavy chain comprises at least one CDR of:
SEQ ID NO.: 6, SEQ ID NO.: 7 or SEQ ID NO.: 8
and wherein the light chain comprises at least one CDR of:
   SEQ ID NO.: 9, KVS or SEQ ID NO.: 10.

The amount of DPP3 protein and/ or DPP3 activity in a sample of bodily fluid of said subject may be determined by different methods, e.g. immunoassays, acitivity assays, mass spectrometric methods etc.

The amount of DPP3 protein and/ or DPP3 activity in a sample of bodily fluid of said subject may be determined for example by one of the following methods:

### 1. Luminescence immunoassay for the quantification of DPP3 protein concentrations (LIA) (Rehfeld et al. 2019 JALM 3(6):943-953).

The LIA is a one-step chemiluminescence sandwich immunoassay that uses white high-binding polystyrene microtiter plates as solid phase. These plates are coated with monoclonal anti-DPP3 antibody AK2555 (capture antibody). The tracer anti-DPP3 antibody AK2553 is labeled with MA70-acridinium-NHS-ester and used at a concentration of 20 ng per well. Twenty microliters of samples (e.g. serum, heparin-plasma, citrate-plasma or EDTA-plasma derived from patients' blood) and calibrators are pipetted into coated white microtiter plates. After adding the tracer antibody AK2553, the microtiter plates are incubated for 3 h at room temperature and 600 rpm. Unbound tracer is then removed by 4 washing steps (350 µL per well). Remaining chemiluminescence is measured for 1s per well by using a microtiter plate luminometer. The concentration of DPP3 is determined with a 6-point calibration curve. Calibrators and samples are preferably run in duplicate.

### 2. Enzyme capture activity assay for the quantification of DPP3 activity (ECA) (Rehfeld et al. 2019 JALM 3(6):943-953).

The ECA is a DPP3-specific activity assay that uses black high-binding polystyrene microtiter plates as solid phase. These plates are coated with monoclonal anti-DPP3 antibody AK2555 (capture antibody). Twenty microliters of samples (e.g. serum, heparin-plasma, citrate-plasma, EDTA-plasma, cerebrospinal fluid and urine) and calibrators are pipetted into coated black microtiter plates. After adding assay buffer (200 µL), the microtiter plates are incubated for 2 h at 22°C and 600 rpm. DPP3 present in the samples is immobilized by binding to the capture antibody. Unbound sample components are removed by 4 washing steps (350 µL per well). The specific activity of immobilized DPP3 is measured by the addition of the fluorogenic substrate, Arg-Arg-β-Naphthylamide (Arg2-βNA), in reaction buffer followed by incubation at 37 °C for 1 h. DPP3 specifically cleaves Arg2-βNA into Arg-Arg dipeptide and fluorescent β-naphthylamine. Fluorescence is measured with a fluorometer using an excitation wavelength of 340 nm and emission is detected at 410 nm. The activity of DPP3 is determined with a 6-point calibration curve. Calibrators and samples are preferably run in duplicates.

### 3. Liquid-phase assay for the quantification of DPP3 activity (LAA) (modified from Jones et al., Analytical Biochemistry, 1982).

The LAA is a liquid phase assay that uses black non-binding polystyrene microtiter plates to measure DPP3 activity. Twenty microliter of samples (e.g. serum, heparin-plasma, citrate-plasma) and calibrators are pipetted into non-binding black microtiter plates. After addition of fluorogenic substrate, Arg2-βNA, in assay buffer (200 µL), the initial βNA fluorescence (T=0) is measured in a fluorimeter using an excitation wavelength of 340 nm and emission is detected at 410 nm. The plate is then incubated at 37 °C for 1 hour. The final fluorescence of (T=60) is measured. The difference between final and initial fluorescence is calculated. The activity of DPP3 is determined with a 6-point calibration curve. Calibrators and samples are preferably run in duplicates.

A variety of immunoassays are known and may be used for the assays and methods of the present invention, these include: radioimmunoassays ("RIA"), homogeneous enzyme- multiplied immunoassays ("EMIT"), enzyme linked immunoadsorbent assays ("ELISA"), apoenzyme reactivation immunoassay ("ARIS"), chemiluminescence- and fluorescence-immunoassays, Luminex-based bead arrays, protein microarray assays, and rapid test formats such as for instance immunochromatographic strip tests ("dipstick immunoassays") and immuno-chromotography assays.

In one embodiment of the invention such an assay is a sandwich immunoassay using any kind of detection technology including but not restricted to enzyme label, chemiluminescence label, electrochemiluminescence label, preferably a fully automated assay. In one embodiment of the invention such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, Biomerieux Vidas^{®}, Alere Triage^{®}.

In one embodiment of the invention it may be a so-called POC-test (point-of-care), that is a test technology which allows performing the test within less than 1 hour near the patient without the requirement of a fully automated assay system. One example for this technology is the immunochromatographic test technology.

In a specific embodiment of the invention said POC-test combines the measurement of more than one analyte at the same time. In another specific embodiment of the invention said analyte is selected from the group of DPP3 and Pro-adrenomedullin or fragments thereof. In a very specific embodiment of the invention said POC-test combines the measurement of DPP3 and mature ADM.

In one embodiment of the invention at least one of the binders is labeled in order to be detected.

In a preferred embodiment said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person *(*The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10. PMID: 16376134*).*

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in (Kirk-Othmer, Encyclopedia of chemical technology, 4th ed., executive editor, J. 1. Kroschwitz; editor, M. Howe-Grant, John Wiley & Sons, 1993, vol.15, p. 518-562, incorporated herein by reference, including citations on pages 551-562). Preferred chemiluminescent dyes are acridiniumesters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

DPP3 activity can be measured by detection of cleavage products of DPP3 specific substrates. Known peptide hormone substrates include Leu-enkephalin, Met-enkephalin, endomorphin 1 and 2, valorphin, β-casomorphin, dynorphin, proctolin, ACTH (Adrenocorticotropic hormone) and MSH (melanocyte-stimulating hormone; *Abramićet al. 2000, Baršun et al. 2007, Dhanda et al.* The cleavage of mentioned peptide hormones as well as other untagged oligopeptides (e.g. Ala-Ala-Ala-Ala, *Dhanda et al. 2008)* can be monitored by detection of the respective cleavage products. Detection methods include, but are not limited to, HPLC analysis (e.g. *Lee & Snyder 1982*)*,* mass spectrometry (e.g. *Abramićet al. 2000*)*,* H1-NMR analysis (e.g. *Vandenberg et al. 1985*)*,* capillary zone electrophoresis (CE; e.g. *Baršun et al. 2007*)*,* thin layer chromatography (e.g. *Dhanda et al. 2008)* or reversed phase chromatography (e.g. *Mazocco et al. 2006*).

Detection of fluorescence due to hydrolysis of fluorogenic substrates by DPP3 is a standard procedure to monitor DPP3 activity. Those substrates are specific di- or tripeptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) coupled to a fluorophore. Fluorophores include but are not limited to β-naphtylamide (2-naphtylamide, βNA, 2NA), 4-methoxy-β-naphtylamide (4-methoxy-2-naphtylamide) and 7-amido-4-methylcoumarin (AMC, MCA; *Abramićet al. 2000, Ohkubo et al.* Cleavage of these fluorogenic substrates leads to the release of fluorescent β-naphtylamine or 7-amino-4-methylcoumarin respectively. In a liquid phase assay or an ECA substrate and DPP3 are incubated in for example a 96 well plate format and fluorescence is measured using a fluorescence detector (*Ellis & Nuenke 1967*)*.* Additionally, DPP3 carrying samples can be immobilized and divided on a gel by electrophoresis, gels stained with fluorogenic substrate (e.g. Arg-Arg-βNA) and Fast Garnet GBC and fluorescent protein bands detected by a fluorescence reader *(Ohkubo et al. 1999).* The same peptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) can be coupled to chromophores, such as p-nitroanilide diacetate. Detection of color change due to hydrolysis of chromogenic substrates can be used to monitor DPP3 activity.

Another option for the detection of DPP3 activity is a Protease-Glo^{™} Assay (commercially available at Promega). In this embodiment of said method DPP3 specific di- or tripeptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) are coupled to aminoluciferin. Upon cleavage by DPP3, aminoluciferin is released and serves as a substrate for a coupled luciferase reaction that emits detectable luminescence.

In a preferred embodiment DPP3 activity is measured by addition of the fluorogenic substrate Arg-Arg-βNA and monitoring fluorescence in real time.

In a specific embodiment of said method for determining active DPP3 in a bodily fluid sample of a subject said capture binder reactive with DPP3 is immobilized on a solid phase.

The test sample is passed over the immobile binder, and DPP3, if present, binds to the binder and is itself immobilized for detection. A substrate may then be added, and the reaction product may be detected to indicate the presence or amount of DPP3 in the test sample. For the purposes of the present description, the term "solid phase" may be used to include any material or vessel in which or on which the assay may be performed and includes, but is not limited to: porous materials, nonporous materials, test tubes, wells, slides, agarose resins (e.g. Sepharose from GE Healthcare Life Sciences), magnetic particals (e.g. Dynabeads^{™} or Pierce^{™} magnetic beads from Thermo Fisher Scientific), etc.

Binders of protein or peptide origin (e.g. antibody, antibody fragments, non-Ig scaffold) are immobilized onto the solid phase by methods comprising: physical adsorption (e.g. by electrostatic interaction or hydrophobic interaction), bioaffinity immobilization (e.g. avidin-biotin, protein A/ G/ L, His-tag and Ni²⁺-NTA, GST-tag and gluthatione, DNA hybridization, aptamers), covalent bond (e.g. amine and N-hydroxysuccinimide) or a combination of said immobilization methods (*Kim & Herr 2013*)*.* Binders of oligonucleotide origin (e.g. aptamers) may be immobilized onto the solid phase by utilization of the (strept)avidin-biotin system (*Müller et al. 2012, Deng et al. 2014*)*.*

In a specific embodiment of said method for determining DPP3 activity in a bodily fluid sample of a subject said separation step is a washing step that removes ingredients of the sample that are not bound to said capture-binder from the captured DPP3. That separation step can be any other step that separates DPP3 bound to said capture-binder from the ingredients of said bodily fluid sample.

In a specific embodiment of said method for determining DPP3 activity in a bodily fluid sample of a subject said DPP3 substrate conversion by immobilized DPP3 is measured (detected) by a method selected from the group comprising: fluorescence of fluorogenic substrates (e.g. Arg-Arg-βNA, Arg-Arg-AMC), color change of chromogenic substrates, luminescence of substrates coupled to aminoluciferin (Promega Protease-Glo^{™} Assay), mass spectrometry, HPLC/ FPLC (reversed phase chromatography, size exclusion chromatography), thin layer chromatography, capillary zone electrophoresis, gel electrophoresis followed by activity staining (immobilized, active DPP3) or western blot (cleavage products).

In a specific embodiment of said method for determining DPP3 acitivity in a bodily fluid sample of a subject said substrate may be selected from the group comprising: Leu-enkephalin, Met-enkephalin, endomorphin 1 and 2, valorphin, β-casomorphin, dynorphin, proctolin, ACTH and MSH, or di- and tri-peptides coupled to a fluorophore, a chromophore or aminoluciferin (Promega Protease-Glo^{™} Assay). Di- or tripeptides that are cleaved by DPP3 include, but are not limited to, Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe. Fluorophores include but are not limited to β-naphtylamide (2-naphtylamide, βNA, 2NA), 4-methoxy-β-naphtylamide (4-methoxy-2-naphtylamide) and 7-amido-4-methylcoumarin (AMC, MCA; Abramic et al. 2000, Ohkubo et al. 1999). Cleavage of these fluorogenic substrates leads to the release of fluorescent β-naphtylamine or 7-amino-4-methylcoumarin respectively. Chromophores include but are not limited to p-nitroanilide diacetate (pNA). The hydrolysis of a peptide-pNA bond in the chromogenic substrates results in the release of pNA which in turn changes color. Thus, the change in absorbance (DA/min) is directly proportional to the enzymatic activity. Using the Protease-Glo^{™} Assay from Promega, upon cleavage by DPP3, aminoluciferin is released and serves as a substrate for a coupled luciferase reaction that emits detectable luminescence.

Subject-matter of the present invention is also a method for prognosing an outcome and/or the risk of an adverse event in a subject that has developed refractory shock, wherein said method is comprising the steps:
- determining the level of DPP3 in a sample of bodily fluid of said subject;
- comparing said level of determined DPP3 to a predetermined threshold,
- correlating said level of DPP3 with said risk of an adverse event in said subject, wherein an elevated level above a certain threshold is predictive for an enhanced risk of said adverse events or,
- correlating said level of DPP3 with success of a therapy or intervention in said subject, wherein a level below a certain threshold is predictive for a success of therapy or intervention.

In the context of the present invention, the term "prognosis" denotes a prediction of how a patient's medical condition will progress. This may include an estimation of the chance of recovery or the chance of an adverse event for said patient. Adverse event is defined as organ dysfunction or mortality. Organ dysfunction is defined as renal decline, cardiac dysfunction or liver dysfunction.

Further embodiments of the present invention are:
1. A method for predicting or diagnosing a refractory shock in a subject that either runs into shock or that has developed shock, wherein said method is comprising the steps:
   - determining the level of DPP3 in a sample of bodily fluid of said subject;
   - comparing said level of determined DPP3 to a predetermined threshold,
   wherein said subject is predicted to run into refractory shock or is diagnosed as having refractory shock if said determined level of DPP3 is above said predetermined threshold.
2. A method for predicting or diagnosing a refractory shock in a subject that either runs into shock or that has developed shock according to embodiment 1, wherein said shock is selected from the group comprising shock due to hypovolemia, cardiogenic shock, obstructive shock and distributive shock, in particular cardiogenic shock or septic shock.
3. A method for predicting or diagnosing a refractory shock in a subject that either runs into shock or that has developed shock according to embodiment 1 and 2, wherein
   - in case of cardiogenic shock said subject may have suffered an acute coronary syndrome (e.g. acute myocardial infarction) or wherein said subject has heart failure (e.g. acute decompensated heart failure), myocarditis, arrhythmia, cardiomyopathy, valvular heart disease, aortic dissection with acute aortic stenosis, traumatic chordal rupture or massive pulmonary embolism or
   - in case of hypovolemic shock said subject may have suffered a hemorrhagic disease including gastrointestinal bleed, trauma, vascular etiologies (e.g. ruptured abdominal aortic aneurysm, tumor eroding into a major blood vessel) and spontaneous bleeding in the setting of anticoagulant use or a non-hemorrhagic disease including vomiting, diarrhea, renal loss, skin losses/insensible losses (e.g. burns, heat stroke) or third-space loss in the setting of pancreatitis, cirrhosis, intestinal obstruction, trauma. or
   - in case of obstructive shock said patient may have suffered a cardiac tamponade, tension pneumothorax, pulmonary embolism or aortic stenosis, or
   - in case of distributive shock said patient may have septic shock, neurogenic shock, anaphylactic shock or shock due to adrenal crisis.
4. A method for predicting or diagnosing a refractory shock in a subject that either runs into shock or that has developed shock according to embodiments 1 to 3, wherein said method is used for initiation and/or termination and/ or stratification and/or guidance of treatment.
5. A method for predicting or diagnosing a refractory shock in a subject that either runs into shock or that has developed shock according to any of embodiments 1 to 4, wherein a treatment is initiated and/or maintained and/or withheld and/ or terminated if said determined level of DPP3 is above said predetermined threshold.
6. A method for predicting or diagnosing a refractory shock in a subject that either runs into shock or that has developed shock according to embodiment 5, wherein said treatment is selected from the group of vasopressors, Angiotensin-Receptor-Agonists and/or precursors thereof, inhibitors of the DPP3 activity and anti-adrenomedullin antibodies or anti-adrenomedullin antibody fragments.
7. A method for predicting or diagnosing a refractory shock in a subject that either runs into shock or that has developed shock according to any of embodiments 1 to 6, wherein either the level of DPP3 protein and/or the level of active DPP3 is determined and compared to a predetermined threshold.
8. A method for predicting or diagnosing a refractory shock in a subject that either runs into shock or that has developed shock according to any of embodiments 1 to 7, wherein the level of DPP3 is determined by contacting said sample of bodily fluid with a capture binder that binds specifically to DPP3.
9. A method for predicting or diagnosing a refractory shock in a subject that either runs into shock or that has developed shock according to embodiment 8, wherein said capture binder for determining the level of DPP3 may be selected from the group of antibody, antibody fragment or non-IgG scaffold.
10. A method for predicting or diagnosing a refractory shock in a subject that either runs into shock or that has developed shock according to embodiments 8 to 9, wherein said capture binder is an antibody.
11. A method for predicting or diagnosing a refractory shock in a subject that either runs into shock or that has developed shock according to any of embodiments 8 to 10, wherein said capture binder is a monoclonal antibody.
12. A method for predicting or diagnosing a refractory shock in a subject that either runs into shock or that has developed shock according to any of embodiments 1 to 11, wherein said sample of bodily fluid is selected from the group of whole blood, plasma, and serum.
13. A method for predicting or diagnosing a refractory shock in a subject that either runs into shock or that has developed shock according to any of embodiments 1 to 12, wherein said method of diagnosing or predicting is conducted at least twice.
14. A method for predicting or diagnosing a refractory shock in a subject that either runs into shock or that has developed shock according to any of embodiments 1 to 13, wherein a treatment with Angiotensin-Receptor-Agonists and/ or precursors thereof and/or inhibitors of the DPP3 activity is initiated and/or continued when the level of DPP3 in said sample is above a certain threshold and/or wherein a treatment with vasopressors is withheld and/ or terminated if said determined level of DPP3 is above said predetermined threshold.
15. A method for predicting or diagnosing a refractory shock in a subject that either runs into shock or that has developed shock according to any of embodiments 1 to 13, wherein a treatment with vasopressors is initiated and/or continued when the level of DPP3 in said sample is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.
16. A method for predicting or diagnosing a refractory shock in a subject that either runs into shock or that has developed shock according to embodiments 14 to 15, wherein in addition the level of Pro-adrenomedullin or fragments thereof is determined and wherein treatment with an anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
17. A method for predicting or diagnosing a refractory shock in a subject that either runs into shock or that has developed shock according to embodiment 14, wherein treatment with an anti-ADM antibody or anti-ADM antibody fragment and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.
18. Vasopressor for use in therapy of shock in a subject that either runs into shock or that has developed shock, wherein said subject has a level of DPP3 in a sample of bodily fluid of said subject that is below a predetermined threshold, when determined by a method according to any of embodiments 1 - 17.
19. Vasopressor for use in therapy of shock in a subject that either runs into shock or that has developed shock according to embodiment 18, wherein said vasopressor is selected from the group comprising dopamine, norepinephrine, a norepinephrine equivalent, epinephrine, phenylephrine and vasopressin.
20. Vasopressor for use in therapy of shock in a subject that either runs into shock or that has developed shock according to embodiment 18 or 19, wherein said vasopressor is administered to said subject in a pharmaceutical formulation.
21. Vasopressor for use in therapy of shock in a subject that either runs into shock or that has developed shock according to any of embodiments 18 to 20, wherein said subject has a blood pressure of equal or less than 65mm Hg.
22. Inhibitor of the activity of DPP3 for use in therapy of shock in a subject that either runs into shock or that has developed shock, wherein said subject has a level of DPP3 in a sample of bodily fluid of said subject that is above a predetermined threshold when determined by a method according to any of embodiments 1 - 17.
23. Inhibitor of the activity of DPP3 for use in therapy of shock in a subject that either runs into shock or that has developed shock according to embodiment 22, wherein the inhibitor of the activity of DPP3 is selected from the group comprising anti-DPP3 antibody or anti-DPP3 antibody fragment or anti-DPP3 non-Ig scaffold.
24. Inhibitor of the activity of DPP3 for use in therapy of shock in a subject that either runs into shock or that has developed shock according to embodiments 22 and 23, wherein said inhibitor has a minimum binding affinity to DPP3 of equal or less than 10⁻⁷ M.
25. Inhibitor of the activity of DPP3 for use in therapy of shock in a subject that either runs into shock or that has developed shock according to any of embodiments 22 to 24, wherein the inhibitor of the activity of DPP3 is an antibody.
26. Inhibitor of the activity of DPP3 for use in therapy of shock in a subject that either runs into shock or that has developed shock according to any of embodiments 22 to 25, wherein said inhibitor is an antibody that is monoclonal.
27. Inhibitor of the activity of DPP3 for use in therapy of shock in a subject that either runs into shock or that has developed shock according to any of embodiments 22 to 26, wherein said inhibitor is a monoclonal antibody, wherein the complementarity determining regions (CDR's) in the heavy chain comprises the sequences: SEQ ID NO.: 7, SEQ ID NO.: 8 and/ or SEQ ID NO.: 9 and the complementarity determining regions (CDR's) in the light chain comprises the sequences: SEQ ID NO.: 10, KVS and/or SEQ ID NO.: 11.
28. Inhibitor of the activity of DPP3 for use in therapy of shock in a subject that either runs into shock or that has developed shock according to any of embodiments 22 to 27, wherein said inhibitor is a humanized monoclonal antibody or humanized monoclonal antibody fragment, wherein the heavy chain comprises the sequence: SEQ ID NO.: 12 and wherein the light chain comprises the sequence: SEQ ID NO.: 13.
29. Inhibitor of the activity of DPP3 for use in therapy of shock in a subject that either runs into shock or that has developed shock according to any of embodiments 22 to 28, wherein said inhibitor is administered in combination with an Angiotensin-Receptor-Agonist and/or precursor thereof.
30. Inhibitor of the activity of DPP3 for use in therapy of shock in a subject that either runs into shock or that has developed shock according to embodiment 29, wherein said Angiotensin-Receptor-Agonist and/ or precursor thereof is selected from the group comprising angiotensin I, angiotensin II, angiotensin III, angiotensin IV, in particular angiotensin II.
31. Angiotensin-Receptor-Agonists and/ or precursors thereof and/or inhibitors of the DPP3 activity for use in the treatment of shock in a subject that either runs into shock or that has developed shock, wherein the treatment with said Angiotensin-Receptor-Agonists and/ or precursors thereof and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold.
32. Vasopressor for use in the treatment of shock in a subject that either runs into shock or that has developed shock, wherein the treatment with said vasopressor is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.
33. Vasopressor for use in the treatment of shock in a subject that either runs into shock or that has developed shock according to embodiment 32, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
34. Angiotensin-Receptor-Agonists and/ or precursors thereof and/or inhibitors of the DPP3 activity for use in the treatment of shock in a subject that either runs into shock or that has developed shock according to embodiment 31, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
35. Anti-ADM antibody or anti-ADM antibody fragment for use in the treatment of shock in a subject that either runs into shock or that has developed shock according to embodiments 33-34, wherein treatment with said anti-ADM antibody or anti-ADM antibody and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.
36. A method of treatment of shock in a subject that either runs into shock or that has developed shock, the method comprising administering vasopressor to said subject, wherein said subject has a level of DPP3 in a sample of bodily fluid of said subject that is below a predetermined threshold, when determined by a method according to any of embodiments 1 - 17.
37. A method of treatment of shock in a subject that either runs into shock or that has developed shock according to embodiment 35, the method comprising administering vasopressor to said subject,
   wherein said vasopressor is selected from the group comprising dopamine, norepinephrine, a norepinephrine equivalent, epinephrine, phenylephrine and vasopressin.
38. A method of treatment of shock in a subject that either runs into shock or that has developed shock according to embodiments 35-36, the method comprising administering vasopressor to said subject, wherein said vasopressor is administered to said subject in a pharmaceutical formulation.
39. A method of treatment of shock in a subject that either runs into shock or that has developed shock according to embodiments 35-37, the method comprising administering vasopressor to said subject,
   wherein said subject has a blood pressure of equal or less than 65mm Hg.
40. Method of treatment of shock in a subject that either runs into shock or that has developed shock, the method comprising administering inhibitor of DPP3 activity to said subject, wherein said subject has a level of DPP3 in a sample of bodily fluid of said subject that is above a predetermined threshold when determined by a method according to any of embodiments 1 - 17.
41. Method of treatment of shock in a subject that either runs into shock or that has developed shock according to embodiment 39, the method comprising administering an inhibitor of DPP3 activity to said subject, wherein the inhibitor of the activity of DPP3 is selected from the group comprising anti-DPP3 antibody or anti-DPP3 antibody fragment or anti-DPP3 non-Ig scaffold.
42. Method of treatment of shock in a subject that either runs into shock or that has developed shock according to embodiments 39-40, the method comprising administering inhibitor of DPP3 activity to said subject, wherein said inhibitor has a minimum binding affinity to DPP3 of equal or less than 10⁻⁷ M.
43. Method of treatment of shock in a subject that either runs into shock or that has developed shock according to embodiments 39-41, the method comprising administering inhibitor of DPP3 activity to said subject, wherein the inhibitor of the activity of DPP3 is an antibody.
44. Method of treatment of shock in a subject that either runs into shock or that has developed shock according to embodiments 39-42, the method comprising administering inhibitor of DPP3 activity to said subject, wherein said inhibitor is an antibody that is monoclonal.
45. Method of treatment of shock in a subject that either runs into shock or that has developed shock according to embodiments 39-43, the method comprising administering inhibitor of DPP3 activity to said subject, wherein said inhibitor is a monoclonal antibody, wherein the complementarity determining regions (CDR's) in the heavy chain comprises the sequences: SEQ ID NO.: 7, SEQ ID NO.: 8 and/ or SEQ ID NO.: 9 and the complementarity determining regions (CDR's) in the light chain comprises the sequences: SEQ ID NO.: 10, KVS and/or SEQ ID NO.: 11.
46. Method of treatment of shock in a subject that either runs into shock or that has developed shock according to embodiments 39-44, the method comprising administering inhibitor of DPP3 activity to said subject, wherein said inhibitor is a humanized monoclonal antibody or humanized monoclonal antibody fragment, wherein the heavy chain comprises the sequence: SEQ ID NO.: 12 and wherein the light chain comprises the sequence: SEQ ID NO.: 13.
47. Method of treatment of shock in a subject that either runs into shock or that has developed shock according to embodiments 39-45, the method comprising administering an inhibitor of DPP3 activity to said subject, wherein said inhibitor is administered in combination with an Angiotensin-Receptor-Agonist and/ or precursors thereof.
48. Method of treatment of shock in a subject that either runs into shock or that has developed shock according to embodiments 39-46, the method comprising administering inhibitor of DPP3 activity to said subject, wherein said Angiotensin-Receptor-Agonist and/ or precursors thereof is selected from the group comprising angiotensin I, angiotensin II, angiotensin III, angiotensin IV, in particular angiotensin II.
49. Method of treatment of shock in a subject that either runs into shock or that has developed shock according to embodiments 39-47, the method comprising administering Angiotensin-Receptor-Agonists and/ or precursors thereof and/or inhibitor of DPP3 activity to said subject, wherein the treatment with said Angiotensin-Receptor-Agonists and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold.
50. Method of treatment of shock in a subject that either runs into shock or that has developed shock according to embodiments 35-38, the method comprising administering vasopressor to said subject, wherein the treatment with said vasopressors is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.
51. Method of treatment of shock in a subject that either runs into shock or that has developed shock according to embodiment 49, the method comprising administering Angiotensin-Receptor-Agonists and/ or precursors thereof and/or inhibitor of DPP3 activity to said subject, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
52. Method of treatment of shock in a subject that either runs into shock or that has developed shock according to embodiment 50, the method comprising administering vasopressor to said subject, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
53. Method of treatment of shock in a subject that either runs into shock or that has developed shock according to embodiment 51 and 52, the method comprising administering anti-ADM antibody or anti-ADM antibody fragment to said subject, wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
54. Method of treatment of shock in a subject that either runs into shock or that has developed shock according to embodiments 51 to 53, the method comprising administering anti-ADM antibody or anti-ADM antibody fragment to said subject, wherein treatment with said anti-ADM antibody or anti-ADM antibody and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.

With the above context, the following consecutively numbered embodiments provide further specific aspects of the invention:
1. A method for predicting or diagnosing a refractory shock in a subject that either runs into shock or that has developed shock, wherein said method is comprising the steps:
   - determining the level of DPP3 in a sample of bodily fluid of said subject;
   - comparing said level of determined DPP3 to a predetermined threshold,
   wherein said subject is predicted to run into refractory shock or is diagnosed as having refractory shock if said determined level of DPP3 is above said predetermined threshold.
2. A method for predicting or diagnosing a refractory shock in a subject that either runs into shock or that has developed shock according to embodiment1, wherein said shock is selected from the group comprising shock due to hypovolemia, cardiogenic shock, obstructive shock and distributive shock, in particular cardiogenic shock or septic shock.
3. A method for predicting or diagnosing a refractory shock in a subject that either runs into shock or that has developed shock according to embodiment 1 and 2, wherein
   - in case of cardiogenic shock said subject may have suffered an acute coronary syndrome (e.g. acute myocardial infarction) or wherein said subject has heart failure (e.g. acute decompensated heart failure), myocarditis, arrhythmia, cardiomyopathy, valvular heart disease, aortic dissection with acute aortic stenosis, traumatic chordal rupture or massive pulmonary embolism or
   - in case of hypovolemic shock said subject may have suffered a hemorrhagic disease including gastrointestinal bleed, trauma, vascular etiologies (e.g. ruptured abdominal aortic aneurysm, tumor eroding into a major blood vessel) and spontaneous bleeding in the setting of anticoagulant use or a non-hemorrhagic disease including vomiting, diarrhea, renal loss, skin losses/insensible losses (e.g. burns, heat stroke) or third-space loss in the setting of pancreatitis, cirrhosis, intestinal obstruction, trauma. or
   - in case of obstructive shock said patient may have suffered a cardiac tamponade, tension pneumothorax, pulmonary embolism or aortic stenosis, or
   - in case of distributive shock said patient may have septic shock, neurogenic shock, anaphylactic shock or shock due to adrenal crisis.
4. A method for predicting or diagnosing a refractory shock in a subject that either runs into shock or that has developed shock according to embodiments 1 to 3, wherein said method is used for initiation and/or termination and/ or stratification and/or guidance of treatment.
5. A method for predicting or diagnosing a refractory shock in a subject that either runs into shock or that has developed shock according to any of embodiments 1 to 4, wherein a treatment is initiated and/or maintained and/or withheld and/ or terminated if said determined level of DPP3 is above said predetermined threshold.
6. A method for predicting or diagnosing a refractory shock in a subject that either runs into shock or that has developed shock according to embodiment 5, wherein said treatment is selected from the group of vasopressors, Angiotensin-Receptor-Agonists and/or precursors thereof, inhibitors of the DPP3 activity and anti-adrenomedullin antibodies or anti-adrenomedullin antibody fragments.
7. A method for predicting or diagnosing a refractory shock in a subject that either runs into shock or that has developed shock according to any of embodiments 1 to 6, wherein either the level of DPP3 protein and/or the level of active DPP3 is determined and compared to a predetermined threshold, wherein the level of DPP3 is determined by contacting said sample of bodily fluid with a capture binder that binds specifically to DPP3.
8. A method for predicting or diagnosing a refractory shock in a subject that either runs into shock or that has developed shock according to any of embodiments 1 to 7, wherein a treatment with Angiotensin-Receptor-Agonists and/ or precursors thereof and/or inhibitors of the DPP3 activity is initiated and/or continued when the level of DPP3 in said sample is above a certain threshold and/or wherein a treatment with vasopressors is withheld and/ or terminated if said determined level of DPP3 is above said predetermined threshold.
9. A method for predicting or diagnosing a refractory shock in a subject that either runs into shock or that has developed shock according to any of embodiments 1 to 7, wherein a treatment with vasopressors is initiated and/or continued when the level of DPP3 in said sample is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.
10. A method for predicting or diagnosing a refractory shock in a subject that either runs into shock or that has developed shock according to embodiment 8, wherein in addition the level of Pro-adrenomedullin or fragments thereof is determined and wherein treatment with an anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
11. A method for predicting or diagnosing a refractory shock in a subject that either runs into shock or that has developed shock according to embodiment 8, wherein treatment with an anti-ADM antibody or anti-ADM antibody fragment and/or Angiotensin-Receptor-Agonists and/ or precursors thereof and/or inhibitors of the DPP3 activity is initiated and/or continued if the level of Pro-adrenomedullin or fragments thereof in said sample is above a certain threshold and said determined level of DPP3 is above said predetermined threshold of DPP3.
12. Vasopressor for use in therapy of shock in a subject that either runs into shock or that has developed shock, wherein said subject has a level of DPP3 in a sample of bodily fluid of said subject that is below a predetermined threshold, when determined by a method according to any of embodiments 1 - 10.
13. Inhibitor of the activity of DPP3 for use in therapy of shock in a subject that either runs into shock or that has developed shock, wherein said subject has a level of DPP3 in a sample of bodily fluid of said subject that is above a predetermined threshold when determined by a method according to any of embodiments 1 - 10, wherein the inhibitor of the activity of DPP3 is selected from the group comprising anti-DPP3 antibody or anti-DPP3 antibody fragment or anti-DPP3 non-Ig scaffold.
14. Inhibitor of the activity of DPP3 for use in therapy of shock in a subject that either runs into shock or that has developed shock according to embodiment13, wherein said inhibitor is administered in combination with an Angiotensin-Receptor-Agonist and/or precursor thereof.
15. Inhibitor of the activity of DPP3 for use in therapy of shock in a subject that either runs into shock or that has developed shock according to embodiment 14, wherein said Angiotensin-Receptor-Agonist and/ or precursor thereof is selected from the group comprising angiotensin I, angiotensin II, angiotensin III, angiotensin IV, in particular angiotensin II.
16. A method of treatment of shock in a subject that either runs into shock or that has developed shock, the method comprising administering vasopressor to said subject, wherein said subject has a level of DPP3 in a sample of bodily fluid of said subject that is below a predetermined threshold, when determined by a method according to any of embodiments 1 - 10.
17. Method of treatment of shock in a subject that either runs into shock or that has developed shock, the method comprising administering inhibitor of DPP3 activity to said subject, wherein said subject has a level of DPP3 in a sample of bodily fluid of said subject that is above a predetermined threshold when determined by a method according to any of embodiments 1 - 10.
18. Method of treatment of shock in a subject that either runs into shock or that has developed shock according to embodiment 17, the method comprising administering an inhibitor of DPP3 activity to said subject, wherein said inhibitor is administered in combination with an Angiotensin-Receptor-Agonist and/ or a precursor thereof.
19. Method of treatment of shock in a subject that either runs into shock or that has developed shock according to embodiments 16-17, the method comprising administering Angiotensin-Receptor-Agonists and/ or precursors thereof and/or inhibitor of DPP3 activity to said subject, wherein the treatment with said Angiotensin-Receptor-Agonists and/or inhibitors of the DPP3 activity is initiated and/or continued when the level of DPP3 in a sample of said subject is above a certain threshold and/or wherein a treatment with vasopressors is withheld and/ or terminated if said determined level of DPP3 is above said predetermined threshold.
20. Method of treatment of shock in a subject that either runs into shock or that has developed shock according to embodiments 16-17, the method comprising administering vasopressor to said subject, wherein the treatment with said vasopressors is initiated and/or continued when the level of DPP3 in a sample of bodily fluid of said subject is below a certain threshold and/or wherein a treatment with Angiotensin-Receptor-Agonists and/ or precursors thereof and/or inhibitors of the DPP3 activity is withheld and/ or terminated if the said determined level of DPP3 is below said predetermined threshold.
21. Method of treatment of shock in a subject that either runs into shock or that has developed shock according to embodiment 18, the method comprising administering Angiotensin-Receptor-Agonists and/ or precursors thereof and/or inhibitor of DPP3 activity to said subject, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
22. Method of treatment of shock in a subject that either runs into shock or that has developed shock according to embodiment 19, the method comprising administering vasopressor to said subject, and wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
23. Method of treatment of shock in a subject that either runs into shock or that has developed shock according to embodiment 20 and 21, the method comprising administering anti-ADM antibody or anti-ADM antibody fragment to said subject, wherein in addition the level of Pro-adrenomedullin or fragments thereof is determined and wherein treatment with said anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
24. A method for prognosing an outcome and/or the risk of an adverse event in a subject that has developed refractory shock, wherein said method is comprising the steps:
   - determining the level of DPP3 in a sample of bodily fluid of said subject;
   - comparing said level of determined DPP3 to a predetermined threshold,
   - correlating said level of DPP3 with said risk of an adverse event in said subject, wherein an elevated level above a certain threshold is predictive for an enhanced risk of said adverse events or,
   - correlating said level of DPP3 with success of a therapy or intervention in said subject, wherein a level below a certain threshold is predictive for a success of therapy or intervention.

### EXAMPLES

### Example 1 - Methods for the measurement of DPP 3 protein and DPP3 activity

Generation of antibodies and determination DPP3 binding ability: Several murine antibodies were produced and screened by their ability of binding human DPP3 in a specific binding assay (see Table 1).

### Peptides/ conjugates for immunization:

DPP3 peptides for immunization were synthesized, see Table 1, (JPT Technologies, Berlin, Germany) with an additional N-terminal cystein (if no cystein is present within the selected DPP3-sequence) residue for conjugation of the peptides to Bovine Serum Albumin (BSA). The peptides were covalently linked to BSA by using Sulfolink-coupling gel (Perbio-science, Bonn, Germany). The coupling procedure was performed according to the manual of Perbio. Recombinant GST-hDPP3 was produced by USBio (United States Biological, Salem, MA, USA).

### Immunization of mice, immune cell fusion and screening:

Balb/c mice were intraperitoneally (i.p.) injected with 84 µg GST-hDPP3 or 100 µg DPP3-peptide-BSA-conjugates at day 0 (emulsified in TiterMax Gold Adjuvant), 84 µg or 100 µg at day 14 (emulsified in complete Freund's adjuvant) and 42 µg or 50 µg at day 21 and 28 (in incomplete Freund's adjuvant). At day 49 the animal received an intravenous (i.v.) injection of 42 µg GST-hDPP3 or 50 µg DPP3-peptide-BSA-conjugates dissolved in saline. Three days later the mice were sacrificed and the immune cell fusion was performed.

Splenocytes from the immunized mice and cells of the myeloma cell line SP2/0 were fused with 1 ml 50% polyethylene glycol for 30 s at 37°C. After washing, the cells were seeded in 96-well cell culture plates. Hybrid clones were selected by growing in HAT medium [RPMI 1640 culture medium supplemented with 20% fetal calf serum and HAT-Supplement]. After one week, the HAT medium was replaced with HT Medium for three passages followed by returning to the normal cell culture medium.

The cell culture supernatants were primarily screened for recombinant DPP3 binding IgG antibodies two weeks after fusion. Therefore, recombinant GST-tagged hDPP3 (USBiologicals, Salem, USA) was immobilized in 96-well plates (100 ng/ well) and incubated with 50 µl cell culture supernatant per well for 2 hours at room temperature. After washing of the plate, 50 µl / well POD-rabbit anti mouse IgG was added and incubated for 1 h at RT. After a next washing step, 50 µl of a chromogen solution (3,7 mM o-phenylendiamine in citrate/ hydrogen phosphate buffer, 0.012% H₂O₂) were added to each well, incubated for 15 minutes at RT and the chromogenic reaction stopped by the addition of 50 µl 4N sulfuric acid. Absorption was detected at 490 mm.

The positive tested microcultures were transferred into 24-well plates for propagation. After retesting the selected cultures were cloned and re-cloned using the limiting-dilution technique and the isotypes were determined.

### Mouse monoclonal antibody production

Antibodies raised against GST-tagged human DPP3 or DPP3-peptides were produced via standard antibody production methods (*Marx et al. 1997*) and purified via Protein A. The antibody purities were ≥ 90% based on SDS gel electrophoresis analysis.

### Characterization of antibodies - binding to hDPP3 and/ or immunization peptide

To analyze the capability of DPP3/ immunization peptide binding by the different antibodies and antibody clones a binding assay was performed:

### a) Solid phase

Recombinant GST-tagged hDPP3 (SEQ ID NO. 1) or a DPP3 peptide (immunization peptide, SEQ ID NO. 2) was immobilized onto a high binding microtiter plate surface (96-Well polystyrene microplates, Greiner Bio-One international AG, Austria, 1 µg/well in coupling buffer [50 mM Tris, 100 mM NaCl, pH7,8], 1h at RT). After blocking with 5% bovine serum albumin, the microplates were vacuum dried.

### b) Labelling procedure (Tracer)

100 µg (100 µl) of the different antiDPP3 antibodies (detection antibody, 1 mg/ ml in PBS, pH 7.4) were mixed with 10 µl acridinium NHS-ester (1 mg/ml in acetonitrile, InVent GmbH, Germany; EP 0 353 971) and incubated for 30 min at room temperature. Labelled antiDPP3 antibody was purified by gel-filtration HPLC on Shodex Protein 5 µm KW-803 (Showa Denko, Japan). The purified labeled antibody was diluted in assay buffer (50 mmol/l potassium phosphate, 100 mmol/l NaCl, 10 mmol/l Na₂-EDTA, 5 g/l bovine serum albumin, 1 g/l murine IgG, 1 g/l bovine IgG, 50 µmol/l amastatin, 100 µmol/l leupeptin, pH 7.4). The final concentration was approx. 5-7*10⁶ relative light units (RLU) of labelled compound (approx. 20 ng labeled antibody) per 200 µl. acridinium ester chemiluminescence was measured by using a Centro LB 960 luminometer (Berthold Technologies GmbH & Co. KG).

### c) hDPP3 binding assay

The plates were filled with 200 µl of labeled and diluted detection antibody (tracer) and incubated for 2-4 h at 2-8 °C. Unbound tracer was removed by washing 4 times with 350 µl washing solution (20 mM PBS, pH 7.4, 0.1 % Triton X-100). Well-bound chemiluminescence was measured by using the Centro LB 960 luminometer (Berthold Technologies GmbH & Co. KG).

### Characterization of antibodies - hDPP3-inhibition analysis

To analyze the capability of DPP3 inhibition by the different antibodies and antibody clones a DPP3 activity assay with known procedure (Jones et al., 1982) was performed. Recombinant GST-tagged hDPP3 was diluted in assay buffer (25 ng/ ml GST-DPP3 in 50 mM Tris-HCl, pH7,5 and 100 µM ZnCl₂) and 200 µl of this solution incubated with 10 µg of the respective antibody at room temperature. After 1 hour of pre-incubation, fluorogenic substrate Arg-Arg-βNA (20 µl, 2mM) was added to the solution and the generation of free βNA over time was monitored using the Twinkle LB 970 microplate fluorometer (Berthold Technologies GmbH & Co. KG) at 37 °C. Fluorescence of βNA is detected by exciting at 340 nm and measuring emission at 410 nm. Slopes (in RFU/ min) of increasing fluorescence of the different samples are calculated. The slope of GST-hDPP3 with buffer control is appointed as 100 % activity. The inhibitory ability of a possible capture-binder is defined as the decrease of GST-hDPP3 activity by incubation with said capture-binder in percent.

The following table represents a selection of obtained antibodies and their binding rate in Relative Light Units (RLU) as well as their relative inhibitory ability (%; table 1). The monoclonal antibodies raised against the below depicted DPP3 regions, were selected by their ability to bind recombinant DPP3 and/ or immunization peptide, as well as by their inhibitory potential.

All antibodies raised against the GST-tagged, full length form of recombinant hDPP3 show a strong binding to immobilized GST-tagged hDPP3. Also antibodies raised against the SEQ ID NO.: 2 peptide bind to GST-hDPP3. The SEQ ID NO.: 2 antibodies also strongly bind to the immunization peptide.

**Table 1: list of antibodies raised against full-length or sequences of hDPP3 and their ability to bind hDPP3 (SEQ ID NO.: 1) or immunization peptide (SEQ ID NO.: 2) in RLU, as well as the maximum inhibition of recombinant GST-hDPP3.**

| **Sequence number** | **Antigen/ Immunogen** | **hDPP3 region** | **Clone** | **hDPPe binding [RLU]** | **immunization peptide binding [RLU]** | **Max. inhibition of hDPP3** |
|---|---|---|---|---|---|---|
| **SEQ ID NO.: 1** | GST tagged recombinant FL-hDPP3 | 1-737 | 2552 | 3.053.621 | 0 | 65% |
| | | | 2553 | 3.777.985 | 0 | 35% |
| | | | 2554 | 1.733.815 | 0 | 30% |
| | | | 2555 | 3.805.363 | 0 | 25% |
| **SEQ ID NO.: 2** | CETVINPETGEQIQSWYRSGE | 474-493 | 1963 | 141.822 | 2.163.038 | 60% |
| | | | 1964 | 100.802 | 2.041.928 | 60% |
| | | | 1965 | 99.493 | 1.986.794 | 70% |
| | | | 1966 | 118.097 | 1.990.702 | 65% |
| | | | 1967 | 113.736 | 1.909.954 | 70% |
| | | | 1968 | 105.696 | 2.017.731 | 65% |
| | | | 1969 | 82.558 | 2.224.025 | 70% |

The development of a luminescence immunoassay for the quantification of DPP3 protein concentrations (DPP3-LIA) as well as an enzyme capture activity assay for the quantification of DPP3 activity (DPP3-ECA) have been described recently (*Rehfeld et al. 2018. JALM. in press*)*,* which is incorporated here in its entirety by reference.

### Example 2 - DPP3 for prognosis of short-term mortality

DPP3 concentration in plasma of patients with sepsis/ septic shock and cardiogenic shock was determined and related to the short term-mortality of the patients.

### Study Cohort - Sepsis/Septic Shock

In 574 plasma samples from patients of the Adrenomedullin and Outcome in Severe Sepsis and Septic Shock (AdrenOSS) study DPP3 was measured. AdrenOSS is a prospective, observational, multinational study including 583 patients admitted to the intensive care unit with sepsis or septic shock (*Hollinger et al., 2018*). 292 patients were diagnosed with septic shock.

### Study Cohort - Cardiogenic Shock

Plasma samples from 108 patients that were diagnosed with cardiogenic shock were screened for DPP3. Blood was drawn within 6 h from detection of cardiogenic shock. Mortality was followed for 7 days.

### hDPP3 immunoassay:

An immunoassay (LIA) or an activity assays (ECA) detecting the amount of human DPP3 (LIA) or the activity of human DPP3 (ECA), respectively, was used for determining the DPP3 level in patient plasma. Antibody immobilization, labelling and incubation were performed as described in Rehfeld et al. (*Rehfeld et al. 2018*).

### Results

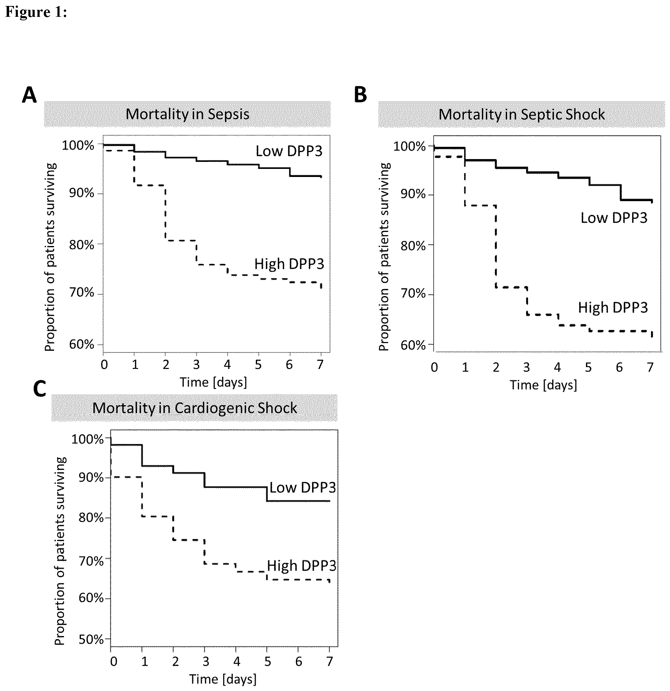

Short-term patients' survival in sepsis patients was related to the DPP3 plasma concentration at admission. Patients with DPP3 plasma concentration above 40.5 ng/mL (3rd Quartile) had an increased mortality risk compared to patients with DPP3 plasma concentrations below this threshold (Figure 1A). Applying this cut-off to the subcohort of septic shock patients, revealed an even more pronounced risk for short-torm mortality in relation to high DPP3 plasma concentrations (Figure 1B). When the same cut-off is applied to patients with cardiogenic shock, also an increased risk for short-term mortality within 7 days is observed in patients with high DPP3 (Figure 1C).

### Example 3 - Purification of human native DPP3

Human erythrocyte lysate was applied on a total of 100 ml of Sepahrose 4B resin (Sigma-Aldrich) and the flow through was collected. The resin was washed with a total of 370 mL PBS buffer, pH 7.4 and the wash fraction was combined with the collected flow through, resulting in a total volume of 2370 mL.

For the immuno-affinity purification step, 110 mg of monoclonal anti-hDPP3 mAb AK2552 were coupled to 25.5 mL of UltraLink Hydrazide Resin (Thermo Fisher Scientific) according to the manufacturer's protocol (GlycoLink Immobilization Kit, Thermo Fisher Scientific). The coupling efficiency was 98%, determined by quantification of uncoupled antibody via Bradford-technique. The resin-antibody conjugate was equilibrated with 10 bed volumes of wash-binding buffer (PBS, 0.1% TritonX-100, pH 7.4), combined with 2370 mL of cleared red blood cell lysate and incubated at 4°C under continuous stirring for 2h. Consequently, 100 mL of the incubation mixture was spread on ten 15 mL polypropylene columns and the flow-through was collected by centrifugation at 1000xg for 30 seconds. This step was repeated several times resulting in 2.5 mL of DPP3-loaded resin per column. Each column was washed 5 times with 10 mL of wash-binding buffer using the gravity-glow approach. DPP3 was eluted by placing each column in 15-mL falcon tube containing 2 mL of neutralization buffer (1M Tris-HCl, pH 8.0), followed by addition of 10 mL of elution buffer (100 mM Glycine-HCl, 0.1% TritonX-100, pH 3.5) per column and immediate centrifugation for 30 seconds at 1000xg. The elution step was repeated 3 times in total resulting in 360 mL of combined eluates. The pH of the neutralized eluates was 8.0.

The combined eluates were loaded on a 5 mL HiTrap Q-sephare HP column (GE Healthcare) equilibrated with IEX-buffer A1 (100 mM Glycine, 150 mM Tris, pH 8.0) using the sample pump of the Äkta Start system (GE Healthcare). After sample loading, the column was washed with five column volumes of IEX Buffer A2 (12 mM NaH₂PO₄, pH 7.4) to remove unbound protein. Elution of DPP3 was achieved by applying a sodium chloride gradient over 10 column volumes (50 mL) in a range of 0 - 1 M NaCl using IEX-buffer B (12 mM NaH₂PO₄,1 M NaCl, pH 7.4). The eluates were collected in 2 mL fractions. Buffers used for ion exchange chromatography were sterile filtered using a 0.22 µM bottle-top filter.

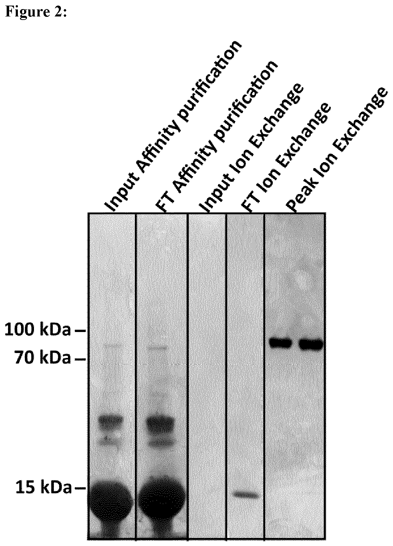

A purification table with the respective yields and activities of each purification step is given in table 2. Figure 2 shows an SDS-PAGE on a gradient gel (4-20%) of native hDPP3 purified from human erythrocyte lysate.

**Table 2: Purification of DPP 3 from human erythrocytes**

| Step | DPP3 amount in % (LIA)^{a)} | Total protein in mg^{b)} | Total activity in µmol/min (ECA)^{c)} | Yield^{d)} in % | Specific activity in U/mg^{e)} | Purification factor^{f)} |
|---|---|---|---|---|---|---|
| Lysate | 100 | 204160 | 55 | 100 | 0.00027 | - |
| IAP | 80.6 | 71.2 | 46.1 | 84 | 0.65 | 2407 |
| IEX | 75 | 6.6 | 38.7 | 70 | 5.9 | 21852 |

| | | | | | | |
|---|---|---|---|---|---|---|
| ^{a)} Relative DPP3 amount was determined in all fractions using the DPP3-LIA assay. Amount of DPP3 in starting material was set to 100% and remaining DPP3 amount in purification fractions was correlated to the starting material. ^{b)} Total protein amount was determined using the method of Lowry modified by Peterson (Peterson 1977. Analytical Biochemistry 356:346-356). ^{c)} Total Arg₂-βNA hydrolyzing activity in µmol of substrate converted per minute was determined using the DPP3-ECA, calibrated via β-naphtylamine (0,05-100 µM). ^{d)} Purification yield was calculated form total Arg₂-βNA hydrolyzing activity. Arg₂-βNA hydrolyzing activity in starting material was set to 100%. ^{e)} Specific activity is defined as µmol of substrate converted per minute and mg of total protein. ^{f)} The purification factor is the quotient of specific activities after and before each purification step. | | | | | | |

### Example 4 - Effect of native DPP3 in an animal model

The effect of native hDPP3 injection in healthy mice was studied by monitoring the shortening fraction and renal resistive index.

Wild type Black 6 mice (8-12 weeks, group size refer to table 3) were acclimated during 2 weeks and a baseline echocardiography was done. The mice were randomly allocated to one of the two groups and, subsequently, native DPP3 protein or PBS were injected intravenously via a retro-orbital injection with a dose of 600 µg/kg for DPP3 protein.

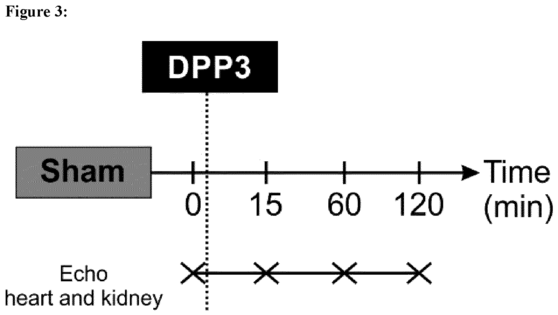

After DPP3 or PBS injection, cardiac function was assessed by echocardiography (*Gao et al. 2011*) and renal function assessed by renal resistive indexi (*Lubas et al., 2014, Dewitte et al, 2012*) at 15, 60 and 120 minutes (Figure 3).

**Table 3: list of experiment groups**

| **Group** | **Number of Animals** | **Treatment** |
|---|---|---|
| WT + PBS | 3 | PBS |
| WT+DPP3 | 4 | Native DPP3 |

### Results

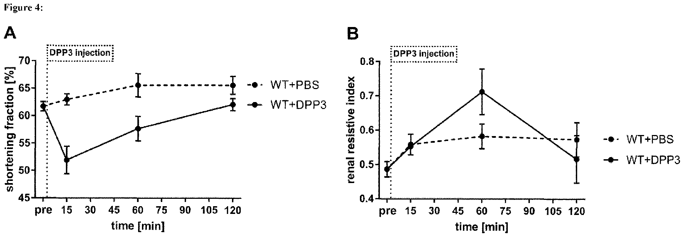

The mice treated with native DPP3 protein show significantly reduced shortening fraction compared to the the control group injected with PBS (Fig 4A). The WT+DPP3 group also displays worsening renal function as observed by the renal resistive index increase (Figure 4B).

### Example 5 - Development of Procizumab

Antibodies raised against SEQ ID NO. 2 were characterized in more detail (epitope mapping, binding affinities, specificity, inhibitory potential). Here the results for clone 1967 of SEQ ID NO.: 2 (AK1967; "Procizumab") are shown as an example.

### Determination of AK1967 epitope on DPP3:

For epitope mapping of AK1967 a number of N- or C-terminally biotinylated peptides were synthesized (PE GmbH, Hennigsdorf, Germany). These peptides include the sequence of the full immunization peptide (SEQ ID No. 2) or fragments thereof, with stepwise removal of one amino acid from either C- or N-terminus (see table 5 for a complete list of peptides).

High binding 96 well plates were coated with 2 µg Avidin per well (Greiner Bio-One international AG, Austria) in coupling buffer (500 mM Tris-HCl, pH 7.8, 100 mM NaCl). Afterwards plates were washed and filled with specific solutions of biotinylated peptides (10 ng/ well; buffer - 1xPBS with 0.5% BSA). Anti-DPP3 antibody AK1967 was labelled with a chemiluminescence label according to Example 1.

The plates were filled with 200 µl of labeled and diluted detection antibody (tracer) and incubated for 4 h at room temperature. Unbound tracer was removed by washing 4 times with 350 µl washing solution (20 mM PBS, pH 7.4, 0.1 % Triton X-100). Well-bound chemiluminescence was measured by using the Centro LB 960 luminometer (Berthold Technologies GmbH & Co. KG). Binding of AK1967 to the respective peptides is determined by evaluation of the relative light units (RLU). Any peptide that shows a significantly higher RLU signal than the unspecific binding of AK1967 is defined as AK1967 binder. The combinatorial analysis of binding and non-binding peptides reveals the specific DPP3 epitope of AK1967.

### Determination of binding affinities using Octet:

The experiment was performed using Octet Red96 (ForteBio). AK1967 was captured on kinetic grade anti-humanFc (AHC) biosensors. The loaded biosensors were then dipped into a dilution series of recombinant GST-tagged human DPP3 (100, 33.3, 11.1, 3.7 nM). Association was observed for 120 seconds followed by 180 seconds of dissociation. The buffers used for the experiment are depicted in table 4. Kinetic analysis was performed using a 1:1 binding model and global fitting.

**Table 4: Buffers used for Octet measurements**

| **Buffer** | **Composition** |
|---|---|
| Assay Buffer | PBS with 0.1% BSA, 0.02% Tween-21 |
| Regeneration Buffer | 10 mM Glycine buffer (pH 1.7) |
| Neutralization Buffer | PBS with 0.1% BSA, 0.02% Tween-21 |

### Western Blot analysis of Binding specificity of AK1967:

Blood cells from human EDTA-blood were washed (3x in PBS), diluted in PBS and lysed by repeated freeze-thaw-cycles. The blood cell lysate had a total protein concentration of 250 µg/ml, and a DPP3 concentration of 10 µg/ml. Dilutions of blood cell lysate (1:40, 1:80, 1:160 and 1:320) and of purified recombinant human His-DPP3 (31.25-500 ng/ml) were subjected to SDS-PAGE and Western Blot. The blots were incubated in 1.) blocking buffer (1xPBS-T with 5% skim milk powder), 2.) primary antibody solution (AK1967 1:2.000 in blocking buffer) and 3.) HRP labelled secondary antibody (goat anti mouse IgG, 1:1.000 in blocking buffer). Bound secondary antibody was detected using the Amersham ECL Western Blotting Detection Reagent and the Amersham Imager 600 UV (both from GE Healthcare).

### DPP3 inhibition assay:

To analyze the capability of DPP3 inhibition by AK1967 a DPP3 activity assay with known procedure (*Jones et al., 1982*) was performed as described in example 1. The inhibitory ability AK1967 is defined as the decrease of GST-hDPP3 activity by incubation with said antibody in percent. The resulting lowered DPP3 activities are shown in an inhibition curve in Figure 1C.

### Epitope mapping:

The analysis of peptides that AK1967 binds to and does not bind to revealed the DPP3 sequence INPETG (SEQ ID No.: 3) as necessary epitope for AK1967 binding (see table 5).

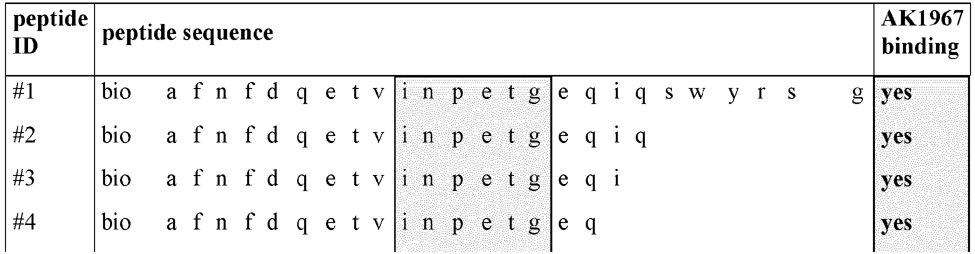

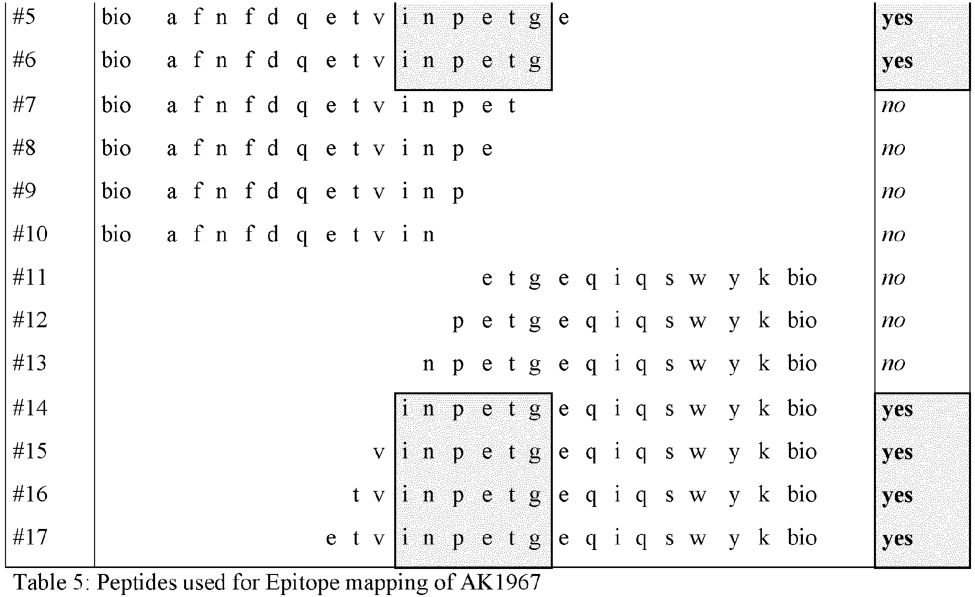

### Binding affinity:

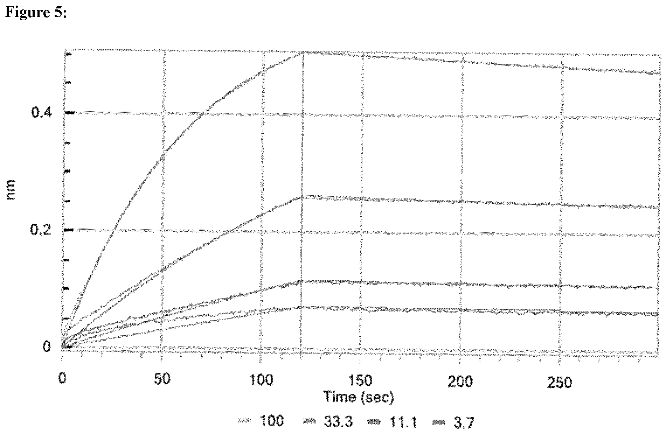

AK1967 binds with an affinity of 2.2*10⁻⁹ M to recombinant GST-hDPP3 (kinetic curves see Figure 5).

### Specificity and inhibitory potential:

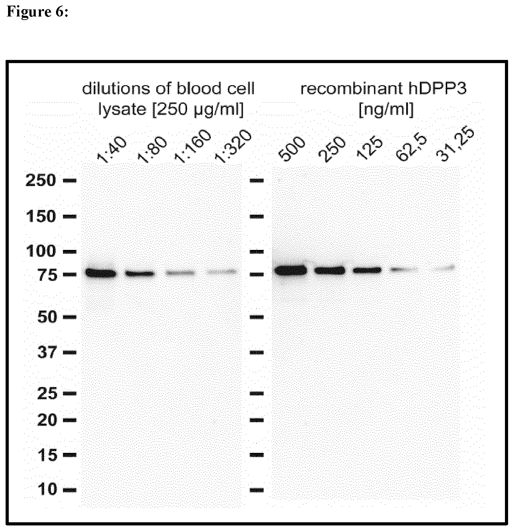

The only protein detected with AK1967 as primary antibody in lysate of blood cells was DPP3 at 80 kDa (Figure 6). The total protein concentration of the lysate was 250 µg/ml whereas the estimated DPP3 concentration is about 10 µg/ml. Even though there is 25 times more unspecific protein in the lysate, AK1967 binds and detects specifically DPP3 and no other unspecific binding takes place.

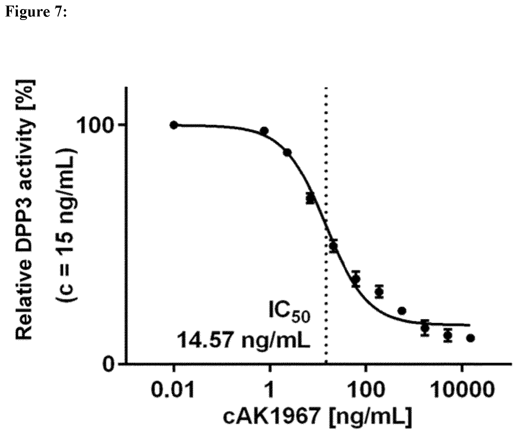

AK1967 inhibits 15 ng/ ml DPP3 in a specific DPP3 activity assay with an IC50 of about 15 ng/ml (Figure 7).

### Chimerization/ Humanization:

The monoclonal antibody AK1967 ("Procizumab"), with the ability of inhibiting DPP3 activity by 70 %, was chosen as possible therapeutic antibody and was also used as template for chimerization and humanization.

### Humanization of murine antibodies may be conducted according to the following procedure:

For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling (Almagro and Fransson, 2008. Humanization of antibodies. Front Biosci. 13:1619-33*).*

With the above context, the variable region can be connected to any subclass of constant regions (IgG, IgM, IgE. IgA), or only scaffolds, Fab fragments, Fv, Fab and F(ab)2. In example 6 and 7 below, the murine antibody variant with an IgG2a backbone was used. For chimerization and humanization a human IgG1κ backbone was used.

For epitope binding, only the CDRs are of importance. The CDRs for the heavy chain and the light chain of the murine anti-DPP3 antibody (AK1967) are shown in SEQ ID No. 6, SEQ ID No. 7 and SEQ ID No. 8 for the heavy chain and SEQ ID No. 9, sequence KVS and SEQ ID No. 10 for the light chain, respectively. Sequencing of the anti-DPP3 antibody (AK1967) revealed an antibody heavy chain variable region (H chain) according to SEQ ID No.: 11 and an antibody light chain variable region (L chain) according to SEQ ID No.: 12.

### Example 6 - Effect of Procizumab in septic shock-induced heart failure

In this experiment, the effect of Procizumab injection in sepsis-induced heart failure rats (Rittirsch et al. 2009) was studied by monitoring the shortening fraction.

### Cecal ligation puncture (CLP) model of septic shock:

Male Wistar rats (2-3 months, 300 to 400 g, group size refers to table 6) from the Centre d'élevage Janvier (France) were allocated randomly to one of three groups. All animals were anesthetized using ketamine hydrochloride (90 mg/ kg) and xylazine (9 mg/ kg) intraperitoneally (i.p.). For induction of polymicrobial sepsis, CLP was performed using Rittirsch's protocol with minor modifications. A ventral midline incision (1.5 cm) was made to allow exteriorization of the cecum. The cecum is then ligated just below the ileocecal valve and punctured once with an 18-gauge needle. The abdominal cavity is then closed in two layers, followed by fluid resuscitation (3 ml/ 100 g body of weight of saline injected subcutaneously) and returning the animal to its cage. Sham animals were subjected to surgery, without getting their cecum punctured. CLP animals were randomized between placebo and therapeutic antibody.

### Study design:

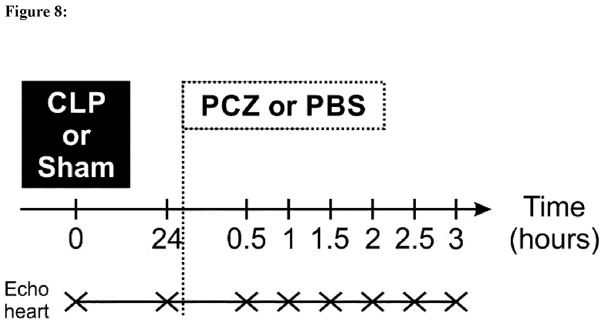

The study flow is depicted in Figure 8. After CLP or sham surgery the animals were allowed to rest for 20 hours with free access to water and food. Afterwards they were anesthetized, tracheotomy done and arterial and venous line laid. At 24 hours after CLP surgery either AK1967 or vehicle (saline) were administered with 5 mg/kg as a bolus injection followed by a 3h infusion with 7.5 mg/kg. As a safety measure, hemodynamics were monitored invasively and continuously from t = 0 till 3 h.

At t=0 (baseline) all CLP animals are in septic shock and developed a decrease in heart function (low blood pressure, low shortening fraction). At this time point Procizumab or vehicle (PBS) were injected (i.v.) and saline infusion was started. There were 1 control group and 2 CLP groups which are summarized in the table below (table 6). At the end of the experiment, the animals were euthanized, and organs harvested for subsequent analysis.

**Table 6: list of experimental groups**

| **Group** | **Number of Animals** | **CLP** | **Treatment** |
|---|---|---|---|
| Sham | 7 | No | PBS |
| CLP-PBS | 6 | Yes | PBS |
| CLP-PCZ | 4 | Yes | PCZ |

### Invasive Blood Pressure:

Hemodynamic variables were obtained using the AcqKnowledge system (BIOPAC Systems, Inc., USA). It provides a fully automated blood pressure analysis system. The catheter is connected to the BIOPAC system through a pressure sensor.

For the procedure, rats were anesthetized (ketamine and xylazine). Animals were moved to the heating pad for the desired body temperature to 37-37.5 °C. The temperature feedback probe was inserted into the rectum. The rats were placed on the operating table in a supine position. The trachea was opened and a catheter (16G) was inserted for an external ventilator without to damage carotid arteries and vagus nerves. The arterial catheter was inserted into the right carotid artery. The carotid artery is separate from vagus before ligation. A central venous catheter was inserted through the left jugular vein allowing administration of PCZ or PBS.

Following surgery, the animals were allowed to rest for the stable condition prior to hemodynamic measurements. Then baseline blood pressure (BP) were recorded. During the data collection, saline infusion via arterial line was stopped.

### Echocardiography:

Animals were anesthetized using ketamine hydrochloride. Chests were shaved and rats were placed in decubitus position. For transthoracic echocardiographic (TTE) examination a commercial GE Healthcare Vivid 7 Ultra-sound System equipped with a high frequency (14-MHz) linear probe and 10-MHz cardiac probe was used. All examinations were recorded digitally and stored for subsequent off-line analysis.

Grey scale images were recorded at a depth of 2 cm. Two-dimensional examinations were initiated in a parasternal long axis view to measure the aortic annulus diameter and the pulmonary artery diameter. M-mode was also employed to measure left ventricular (LV) dimensions and assess fractional shortening (FS%). LVFS was calculated as LV end-diastolic diameter - LV end-systolic diameter / LV end-diastolic diameter and expressed in %. The time of end-diastole was therefore defined at the maximal diameter of the LV. Accordingly, end-systole was defined as the minimal diameter in the same heart cycle. All parameters were measured manually. Three heart cycles were averaged for each measurement.

From the same parasternal long axis view, pulmonary artery flow was recorded using pulsed wave Doppler. Velocity time integral of pulmonary artery outflow was measured.

From an apical five-chamber view, mitral flow was recorded using pulsed Doppler at the level of the tip of the mitral valves.

### Results:

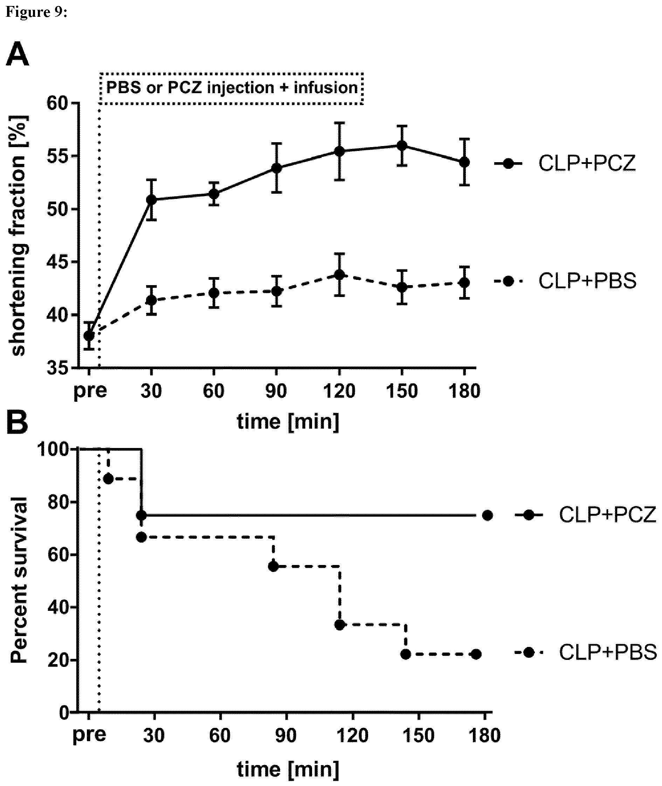

The septic shock-induced heart failure rats treated with PBS (CLP+PBS) show reduced shortening fraction compared to the sham animals (Fig. 9A). The CLP+PBS group also displays high mortality rate (Fig. 9B). In contrast, application of Procizumab to sep-induced heart failure rats improves shortening fraction (Fig. 9A) and drastically reduces the mortality rate (Fig. 9B).

### Example 7 - Effect of Procizumab on heart and kidney function

The effect of Procizumab in isoproterenol-induced heart failure in mice was studied by monitoring the shortening fraction and renal resistive index.

### Isoproterenol-induced cardiac stress in mice:

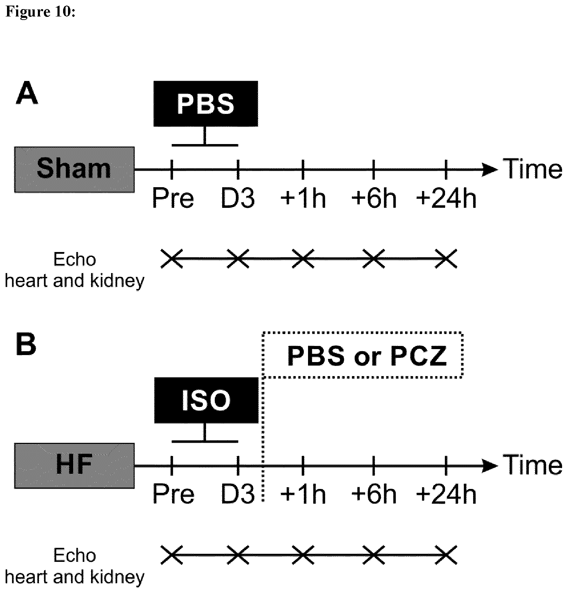

Acute heart failure was induced in male mice at 3 months of age by two daily subcutaneous injections of 300 mg/kg of Isoproterenol, a non-selective β-adrenergic agonist (DL-Isoproterenol hydrochloride, Sigma Chemical Co) (ISO) for two days (*Vergaro et al, 2016*). ISO dilution was performed in NaCl 0.9%. ISO-treated mice were randomly assigned to two groups (Table 7) and PBS or Procizumab (10 mg/kg) were injected intravenously after baseline echocardiography *(Gao et al., 2011)* and renal resistive index measurements (*Lubas et al., 2014, Dewitte et al, 2012)* were performed at day 3 (Figure 10 A and B).

Cardiac function was assessed by echocardiography (Gao et al., 2011) and by the renal resistive index (*Lubas et al., 2014, Dewitte et al, 2012*) at 1 hour, 6 hours and 24 hours (Figure 10 A and B). The group of mice that was injected with vehicle (PBS) instead of isoproterenol was subjected to no further pharmacological treatment and served as the control group (Table 7).

**Table 7: list of experimental groups**

| **Group** | **Number of Animals** | **Treatment** |
|---|---|---|
| Sham+PBS | 27 | PBS |
| HF+PBS | 15 | PBS |
| HF+PCZ | 20 | Procizumab (PCZ) |

### Results:

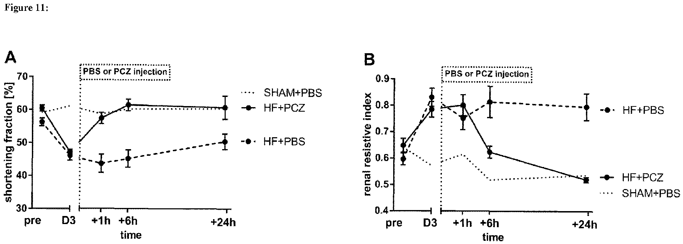

Application of Procizumab to isoproterenol-induced heart failure mice restores heart function within the first hour after administration (Fig. 11A). Kidney function of sick mice shows significant improvement at 6 hours post Procizumab injection and is comparable to the kidney function of sham animals at 24 hours (Fig. 11B).

### Example 8 - DPP3 indicates vasopressor need and response to vasopressor therapy

DPP3 concentrations in plasma of septic shock patients were determined using a hDPP3 immunoassay and related to the need for vasopressor therapy.

### Study Cohort - Septic Shock

Plasma samples from 292 patients that were diagnosed with septic shock from the AdrenOSS (see example 2) study were screened for DPP3. Human DPP3 was measured as described in Example 1.

### Results

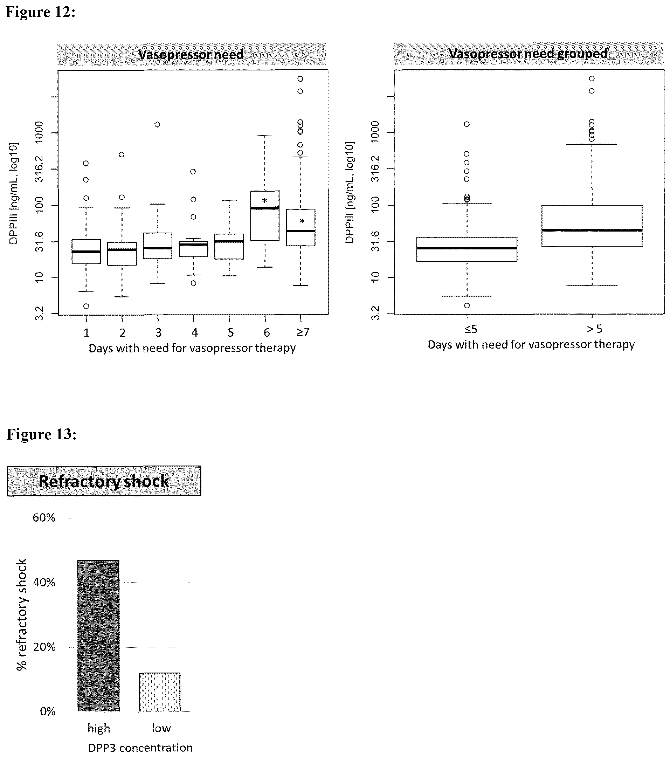

Patients with an increased need to vasopressor administration for more than 5 days had high plasma concentrations of DPP3 (Figure 12A). In contrast, patients that responded to vasopressor administration and where vasopressor administration could be discontinued within the first 5 days, had significantly reduced plasma DPP3 concentrations (Figure 12B). This indicates that development of refractory shock and thus an increased need of vasopressor administration due to a diminished response of this therapy, is related to high DPP3 plasma concentrations in patients with septic shock.

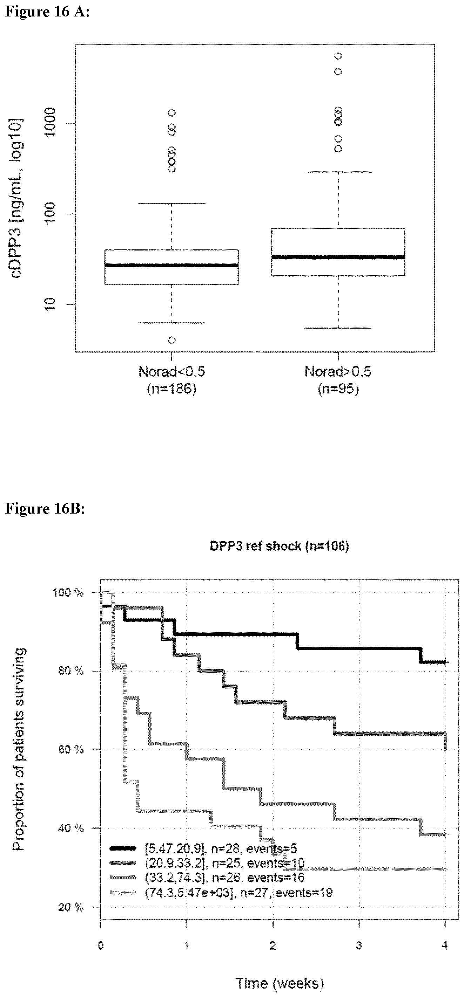

Patients with vasopressor-resistant refractory septic shock (noradrenaline >0.5 µg/kg/min) show significantly higher plasma DPP3 concentrations than compared to patients that required noradrenaline doses of < 0.5 µg/kg/min (p<0.001) (Figure 16A). Moreover, the DPP3 plasma concentration was strongly associated with mortality in patients with vasopressor-resistant refractory septic shock (Figure 16B).

### Example 9 - DPP3 is related to refractory shock

DPP3 concentrations in plasma of cardiogenic shock patients were determined using a hDPP3 immunoassay and related to the development of refractory shock.

### Study Cohort - Cardiogenic Shock

Plasma samples from 57 patients that were diagnosed with cardiogenic shock after acute myocardial infarction were screened for DPP3. Human DPP3 was measured as described in Example 1.

### Results

Patients with a DPP3 plasma concentration above a certain threshold on admission (59.1 ng/ml; 3^{rd} quartile) developed refractory shock to a higher extent (47%) than patients with DPP3 plasma concentrations below 59.1 ng/ml (12%) (Figure 13).

### Example 10 - DPP3 and bio-ADM indicate short-term mortality in shock

DPP3 and bio-ADM concentrations in plasma of septic shock patients were determined using a hDPP3 and a bio-ADM immunoassay and related to the short term-mortality of the patients.

### Study Cohort - Septic Shock

Plasma samples from 292 patients that were diagnosed with septic shock from the AdrenOSS study were screened for DPP3 and bio-ADM. Human DPP3 was measured as described in Example 1. Bio-ADM was measured as decribed in Weber et al. (Weber et al. 2017. JALM 2(2): 1-4).

### Results

Plasma concentrations of bio-ADM and DPP3 were measured in septic schock patients. Patients were grouped according to specific cut-offs determined to be the 3^{rd} quartile of all measured plasma concentrations of the respective marker (Table 8). Equal numbers of patients had either high DPP3 only or high bio-ADM only (15.4%), while a lesser extent showed elevated plasma concentrations in both, bio-ADM and DPP3 (9.6%).

**Table 8: Patient numbers with low/high DPP3 and low/high bio-ADM. Cut-offs were assigned based on the Q3 (highest 25%) for both.**

| | **DPP3 low (< 48.4 ng/ml)** | **DPP3 high (> 48.4 ng/ml)** |
|---|---|---|
| **bio-ADM low (< 213 pg/mL)** | 174 (59.6%) | 45 (15.4%) |
| **bio-ADM high (> 213 pg/mL)** | 45 (15.4%) | 28 (9.6%) |

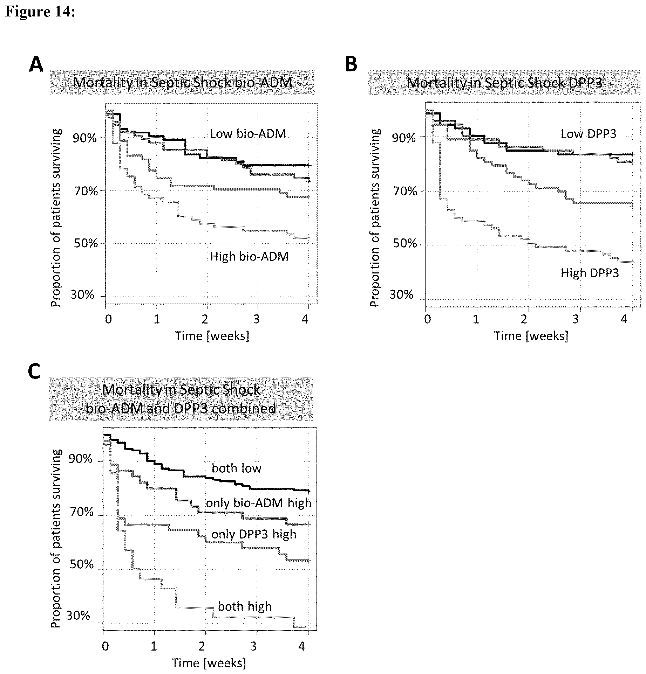

Mortality within the first 4 weeks after admission was related to the bio-ADM and DPP3 concentration on admission. Patients with either only a high bio-ADM or only a high DPP3 plasma concentration had a substantial increased risk to die within the first 4 weeks compared to patients that had a plasma concentration of either bio-ADM (Figure 14A) or either DPP3 (Figure 14B) below a certain threshold (3^{rd} quartile). Survival in patients with high bio-ADM was better than survival in patients with high DPP3.

When both markers, bio-ADM and DPP3, were combined, an even higher risk for short-term mortality was identified in comparison to the mortality that was related to an increase of one of both markers only (Figure 14C). Merely 28.6% of the patients with high bio-ADM and high-DPP3 plasma concentrations survived the first 4 weeks after admission.

### Example 11 - DPP3 and bio-ADM DPP3 indicates vasopressor need and response to vasopressor therapy

DPP3 and bio-ADM concentrations in plasma of septic shock patients were determined using a hDPP3 and a bio-ADM immunoassay and related to the need for vasopressor therapy.

### Study Cohort - Septic Shock

In 292 plasma samples from patients that were diagnosed with septic shock from the AdrenOSS study DPP3 and bio-ADM concentrations were measured. Human DPP3 was measured as described in Example 1. Bio-ADM was measured as decribed in Weber et al. *(*Weber et al. 2017. JALM 2(2): 1-4)*.*

### Results

Plasma concentrations of bio-ADM and DPP3 were measured in septic schock patients. Patients were grouped according to specific cut-offs determined as the 3^{rd} quartile of all measured plasma concentrations of the respective marker in the septic shock cohort (low DPP3 < 48.4 ng/mL, low bio-ADM < 213 pg/mL; high bio-ADM ≥ 213 pg/mL; high DPP3 ≥ 48.4 ng/mL).

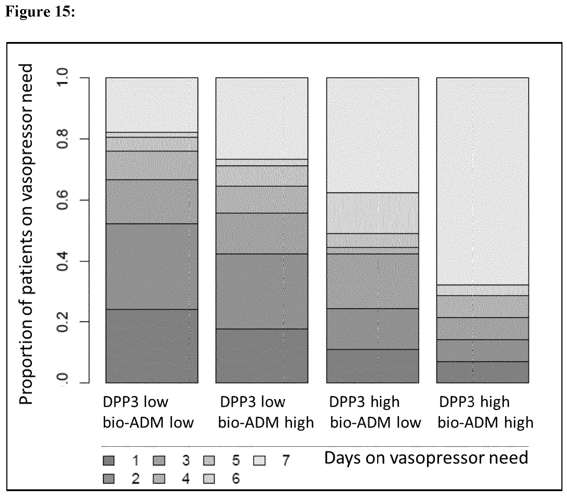

Patients with high DPP3, but low bio-ADM plasma concentrations had a higher need for consecutive vasopressor administration compared to patients with either low DPP3 + low bio-ADM or low DPP3 + high bio-ADM (Figure 15; Table 9). In contrast, patients that had low DPP3 and low bio-ADM plasma concentrations, or patients that had low DPP3, but high bio-ADM plasma concentrations on admission could be discontinued of vasopressor administration earlier than patients with high DPP3 plasma concentrations on admission (Figure 15; Table 9). This indicates that vasopressor therapy in septic shock patients with high bio-ADM can be discontinued earlier due to a better therapeutic response compared to septic shock patients with high DPP3. These patients (high DPP3) need longer treatment with vasopressors due to non-responsiveness.

**Table 9: Patients with vasopressor need grouped according to their DPP3 and bio-ADM plasma concentration at admission. Cut-offs were assigned based on the Q3 (highest 25% of determined values) for both; low DPP3 < 48.4 ng/mL, low bio-ADM < 213 pg/mL; high bio-ADM ≥ 213 pg/mL; high DPP3 ≥ 48.4 ng/mL; 7: ≥7 days on vasopressor or dead within 7 days.**

| **Days with vasopressor need** | **DPP3 Low bioADM low** | **DPP3 low bioADM high** | **DPP3 high bioADMlow** | **DPP3 high bioADM high** |
|---|---|---|---|---|
| **1** | 42 | 8 | 5 | 2 |
| **2** | 49 | 11 | 6 | 2 |
| **3** | 25 | 6 | 8 | 2 |
| **4** | 16 | 4 | 1 | 2 |
| **5** | 8 | 3 | 2 | 0 |
| **6** | 3 | 1 | 6 | 1 |
| **7** | 31 | 12 | 17 | 19 |

### EXAMPLES OF SEQUENCES

**SEQ ID No. 1 - hDPP3 aa 1-737** 
MADTQYILPNDIGVSSLDCREAFRLLSPTERLYAYHLSRAAWYGGLAVLLQTSPEAP
YIYALLSRLFRAQDPDQLRQHALAEGLTEEEYQAFLVYAAGVYSNMGNYKSFGDTK
FVPNLPKEKLERVILGSEAAQQHPEEVRGLWQTCGELMFSLEPRLRHLGLGKEGITTY
FSGNCTMEDAKLAQDFLDSQNLSAYNTRLFKEVDGEGKPYYEVRLASVLGSEPSLDS
EVTSKLKSYEFRGSPFQVTRGDYAPILQKVVEQLEKAKAYAANSHQGQMLAQYIESF
TQGSIEAHKRGSRFWIQDKGPIVESYIGFIESYRDPFGSRGEFEGFVAVVNKAMSAKFE
RLVASAEQLLKELPWPPTFEKDKFLTPDFTSLDVLTFAGSGIPAGINIPNYDDLRQTEG
FKNVSLGNVLAVAYATQREKLTFLEEDDKDLYILWKGPSFDVQVGLHELLGHGSGK
LFVQDEKGAFNFDQETVINPETGEQIQSWYRSGETWDSKFSTIASSYEECRAESVGLY
LCLHPQVLEIFGFEGADAEDVIYVNWLNMVRAGLLALEFYTPEAFNWRQAHMQARF
VILRVLLEAGEGLVTITPTTGSDGRPDARVRLDRSKIRSVGKPALERFLRRLQVLKSTG
DVAGGRALYEGYATVTDAPPECFLTLRDTVLLRKESRKLIVQPNTRLEGSDVQLLEY
EASAAGLIRSFSERFPEDGPELEEILTQLATADARFWKGPSEAPSGQA
**SEQ ID No. 2 - hDPP3 aa 474-493 (N-Cys) - immunization peptide with additional N-terminal Cystein**
   CETVINPETGEQIQSWYRSGE
**SEQ ID No. 3 - hDPP3 aa 477-482 - epitope of AK1967**
   INPETG
**SEQ ID No. 4 - variable region of murine AK1967 in heavy chain** 
QVTLKESGPGILQPSQTLSLTCSFSGFSLSTSGMSVGWIRQPSGKGLEWLAHIWWNDN
KSYNPALKSRLTISRDTSNNQVFLKIASVVTADTGTYFCARNYSYDYWGQGTTLTVS
S
**SEQ ID No. 5 - variable region of murine AK1967 in light chain** 
DVVVTQTPLSLSVSLGDPASISCRSSRSLVHSIGSTYLHWYLQKPGQSPKLLIYKVSNR
FSGVPDRFSGSGSGTDFTLKISRVEAEDLGVYFCSQSTHVPWTFGGGTKLEIK
**SEQ ID No. 6 - CDR1 of murine AK1967 in heavy chain**
   GFSLSTSGMS
**SEQ ID No. 7 - CDR2 of murine AK1967 in heavy chain**
   IWWNDNK
**SEQ ID No. 8 - CDR 3 of murine AK1967 in heavy chain**
   ARNYSYDY
**SEQ ID No. 9 - CDR1 of murine AK1967 in light chain**
   RSLVHSIGSTY
**CDR2 of murine AK1967 in light chain**
   KVS
**SEQ ID No. 10 - CDR3 of murine AK1967 in light chain**
   SQSTHVPWT
**SEQ ID No. 11 - humanized AK1967 - heavy chain sequence (IgG1κ backbone)** 
MDPKGSLSWRILLFLSLAFELSYGQITLKESGPTLVKPTQTLTLTCTFSGFSLSTSGMS
VGWIRQPPGKALEWLAHIWWNDNKSYNPALKSRLTITRDTSKNQVVLTMTNMDPV
DTGTYYCARNYSYDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALH
NHYTQKSLSLSPG
**SEQ ID No. 12 - humanized AK1967 - light chain sequence (IgG1κ backbone)** 
METDTLLLWVLLLWVPGSTGDIVMTQTPLSLSVTPGQPASISCKSSRSLVHSIGSTYLY
WYLQKPGQSPQLLIYKVSNRFSGVPDRFSGSGSGTDFTLKISRVEAEDVGVYYCSQST
HVPWTFGGGTKVEIKRTVAAPSVFIFPPSDEQLKSGTASVVCLLNNFYPREAKVQWK
 
VDNALQSGNSQESVTEQDSKDSTYSLSSTLTLSKADYEKHKVYACEVTHQGLSSPVT
KSFNRGEC
**SEQ ID No. 13 - Angiotensin II (synonyme: 5-isoleucine-angiotensin II)**
   DRVYIHPF
**SEQ ID No. 14 - Angiotensin II analogue (5-valine-angiotensin II)**
   DRVYVHPF
**SEQ ID No. 15 - Angiotensin II analogue (Asn¹-Phe⁴)**
   NRVFIHPF
**SEQ ID No. 16 - Angiotensin II hexapeptide**
   VYIHPF
**SEQ ID No. 17 - Angiotensin II nonapeptide**
   NRVYYVHPF
**SEQ ID No. 18 - Angiotensin II analogue ([Asn¹-Ile⁵-Ile⁸]-angiotensin II)**
   NRVYIHPI
**SEQ ID No. 19 - Angiotensin II analogue ([Asn¹-Ile⁵-Ala⁸]-angiotensin II)**
   NRVYIHPA
**SEQ ID No. 20 - Angiotensin II analogue ([Asn¹-diiodoTyr⁴-Ile⁵]-angiotensin II**
   NRVYIHPF
**SEQ ID No. 21 - Angiotensin III**
   RVYIHPF
**SEQ ID No. 22 - Angiotensin III analogue (Val⁴-angiotensin III)**
   RVYVHPF
**SEQ ID No. 23 - Angiotensin III analogue (Phe³-angiotensin III)**
   RVFIHPF
**SEQ ID No. 24** - **Angiotensin III analogue ([Ile⁴-Ala⁷]-angiotensin III)**
   RVYIHPA
**SEQ ID No. 25 - Angiotensin III analogue (diiodoTyr³-Ile⁴]-angiotensin III)**
   RVYIHPF
**SEQ ID No. 26 - Angiotensin IV**
   VYIHPF
**SEQ ID No. 27** - **Angiotensin IV analogue (Val³-angiotensin IV)**
   VYVHPF
**SEQ ID No. 28 - Angiotensin IV analogue (Phe²-angiotensin IV)**
   VFIHPF
**SEQ ID No. 29** - **Angiotensin IV analogue ([Ile³-Ala⁶]-angiotensin IV)**
   VYIHPA
**SEQ ID No. 30** - **Angiotensin IV analogue ([diiodoTyr²-Ile³-angiotensin IV)**
   VYIHPF
**SEQ ID No. 31 (proADM): 164 amino acids (22 - 185 of preproADM)** 
ARLDVASEF RKKWNKWALS RGKRELRMSS SYPTGLADVK AGPAQTLIRP
QDMKGASRSP EDSSPDAARI RVKRYRQSMN NFQGLRSFGC RFGTCTVQKL
AHQIYQFTDK DKDNVAPRSK ISPQGYGRRR RRSLPEAGPG RTLVSSKPQA
HGAPAPPSGS APHFL
**SEQ ID No. 32 (Proadrenomedullin N-20 terminal peptide, PAMP): amino acids 22 - 41 of preproADM**
   ARLDVASEF RKKWNKWALS R
**SEQ ID No. 33 (Midregional proAdrenomedullin, MR-proADM): amino acids 45 - 92 of preproADM**
   ELRMSS SYPTGLADVK AGPAQTLIRP QDMKGASRSP EDSSPDAARI RV
**SEQ ID No. 34 (mature Adrenomedullin (mature ADM); amidated ADM; bio-ADM; hADM): amino acids 95 - 146 -CONH₂** 
YRQSMN NFQGLRSFGC RFGTCTVQKL AHQIYQFTDK DKDNVAPRSK ISPQGY -
CONH2
**SEQ ID No. 35 (Adrenomedullin 1-52-Gly (ADM 1-52-Gly)): amino acids 95 - 147 of preproADM**
   YRQSMN NFQGLRSFGC RFGTCTVQKL AHQIYQFTDK DKDNVAPRSK ISPQGYG
**SEQ ID No. 36 (C-terminal proAdrenomedullin, CT-proADM): amino acids 148 - 185 of preproADM**
   RRR RRSLPEAGPG RTLVSSKPQA HGAPAPPSGS APHFL
**SEQ ID No. 37 (N-terminal part of mature ADM): amino acids 1-21 of mature ADM**
   YRQSMNNFQGLRSFGCRFGTC
**SEQ ID No. 38 (CDR1 heavy chain anti-ADM antibody)**
   GYTFSRYW
**SEQ ID No. 39 (CDR2 heavy chain anti-ADM antibody)**
   ILPGSGST
**SEQ ID No. 40 (CDR3 heavy chain anti-ADM antibody)**
   TEGYEYDGFDY
**SEQ ID No. 41 (CDR1 light chain anti-ADM antibody)**
   QSIVYSNGNTY
**SEQ ID No. 42 (CDR3 light chain anti-ADM antibody)**
   FQGSHIPYT
**SEQ ID No. 43 (anti-ADM antibody (Adrecizumab) heavy chain)** 
QVQLVQSGAEVKKPGSSVKVSCKASGYTFSRYWIEWVRQAPGQGLEWIGEILPGSGS
TNYNQKFQGRVTITADTSTSTAYMELSSLRSEDTAVYYCTEGYEYDGFDYWGQGTT
VTVSSASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKTHTCPP
CPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK
**SEQ ID NO: 44 (anti-ADM antibody (Adrecizumab) light chain)**

DVVLTQSPLSLPVTLGQPASISCRSSQSIVYSNGNTYLEWYLQRPGQSPRLLIYRVSNR
FSGVPDRFSGSGSGTDFTLKISRVEAEDVGVYYCFQGSHIPYTFGGGTKLEIKRTVAA
PSVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGEC

### FIGURE DESCRIPTION

**Figure 1****:** Kaplan-Meier survival plots in relation to low (< 40.5 ng/mL) and high (≥ 40.5 ng/mL) DPP3 concentrations. (A) 7-Day survival of patients with sepsis in relation to DPP3 plasma concentration; (B) 7-Day survival of Patients with cardiogenic shock in relation to DPP3 plasma concentration; (C) 7-Day survival of patients with septic shock in relation to DPP3 plasma concentration.
**Figure 2****:** SDS-PAGE on a gradient gel (4-20%) of native hDPP3 purified from human erythrocyte lysate. Molecular weight marker is indicated as arrows.
**Figure 3****:** Experimental design - Effect of native DPP3 in an animal model.
**Figure 4****:** (A) DPP3 injection causes shortening fraction reduction and therefore leads to deteriorating heart function. (B) Decreased kidney function is also observed via increased renal resistive index.
**Figure 5****:** Association- and dissociation curve of the AK1967-DPP3 binding analysis using Octet. AK1967 loaded biosensors were dipped into a dilution series of recombinant GST-tagged human DPP3 (100, 33.3, 11.1, 3.7 nM) and association and dissociation monitored.
**Figure 6****:** Western Blot of dilutions of blood cell lysate and detection of DPP3 with AK1967 as primary antibody.
**Figure 7****:** Inhibition curve of native DPP3 from blood cells with inhibitory antibody AK1967. Inhibition of DPP3 by a specific antibody is concentration dependent, with an IC₅₀ at ~15 ng/ml when analyzed against 15 ng/ml DPP3.
**Figure 8****:** Experimental setup - Effect of Procizumab in sepsis-induced heart failure.
**Figure 9****:** Procizumab drastically improves shortening fraction (A) and mortality rate (B) in sepsis-induced heart failure rats.
**Figure 10****:** Experimental design - Isoproterenol-induced cardiac stress in mice followed by Procizumab treatment (B) and control (A).
**Figure 11****:** Procizumab improved shortening fraction (A) and reduced the renal resistive index (B) within 1 hour and 6 hours after administration, respectively, in isoproterenol-induced heart failure mice.
**Figure 12****:** (A) Days with need of vasopressor therapy in septic shock patients in relation to DPP3 plasma concentrations. ≥7 days on vasopressor or dead within 7 days; * p<0.05 in post hoc comparison vs. groups 1-4 (B) Need of vasopressor therapy in septic shock patients in relation to DPP3 plasma concentrations. Patients with vasopressor therapy for maximal 5 days (≤5), and patients with vasopressor therapy longer than 5 days or that died within 7 days were grouped together (>5).
**Figure 13****:** DPP3 is related to refractory shock. High DPP3 concentrations in plasma of cardiogenic shock patients are related with a higher risk to develop refractory cardiogenic shock compared to patients that have DPP3 plasma concentrations below a certain threshold.
**Figure 14****:** Kaplan-Meier survival plots (A) 4-week survival of patients with septic shock in relation to bio-ADM plasma concentration; values are grouped in quartiles (B) 4-week survival of patients with septic shock in relation to DPP3 plasma concentration; values are grouped in quartiles (C) 4-week survival of patients with septic shock in relation to bio-ADM and DPP3 plasma concentration; Cut-offs were determined based on 3^{rd} quartile of all measured plasma concentrations of the respective marker: both low DPP3 < 48.4 ng/mL, bio-ADM < 213 pg/mL; bio-ADM high ≥ 213 pg/mL; DPP3 high ≥ 48.4 ng/mL.
**Figure 15****:** Patients with vasopressor need grouped according to their DPP3 and bio-ADM plasma concentration on admission. Proportions of patients receiving vasopressors for 1-7 days are shown in greyscale - lighter colors represent longer treatment duration. Cut-offs were assigned based on the Q3 (highest 25% of determined values) for both; low DPP3 < 48.4 ng/mL, low bio-ADM < 213 pg/mL; high bio-ADM ≥ 213 pg/mL; high DPP3 ≥ 48.4 ng/mL; 7: ≥7 days on vasopressor or dead within 7 days.
**Figure 16****:** (A) Plasma DPP3 concentration in patients with septic refractory shock who are in need of the vasopressor noradrenalin below (n=186) and above (n=95) 0.5 µg/kg/min (p<0.001). (B) Kaplan-Meier survival plots (A) 4-week survival of patients with septic refractory shock in relation to DPP3 plasma concentration; values are grouped in quartiles.

### REFERENCES

1. Hollenberg et al. Ann Intern Med 1999; 131:47-59.
2. Reynolds HR, Hochman JS. Circulation 2008; 117:686-697.
3. Tarvasmäki et al. Eur J Heart Fail John Wiley & Sons, Ltd; 2018; 20:572-581.
4. Thiele Et al. N Engl J Med 2012; 367:1287-1296.
5. Champion S. Eur J Heart Fail 2018; 20:197-198.
6. Hochman JS. Circulation 2003; 107:2998-3002.
7. Shah et al. Clin Res Cardiol 2018;107:287-303*.*
8. Schmidt et al. Eur Heart J 2015;36:2246- 2256.
9. Muller et al. Intensive Care Med 2016; 42:370-378.
10. Chen et al. Crit Care 2016; 20:336.
11. Harjola et al. Eur J Heart Fail John Wiley & Sons, Ltd; 2015; 17:501-509.
12. Harjola et al. Eur J Heart Fail John Wiley & Sons, Ltd; 2018; 20:1081-1099.
13. Bakker et al. Am J Surg 1996; 171:221-226.
14. Attaná et al. Acute Card Care 2012;14:20-26.
15. Zhang Z, Xu X. Crit Care Med 2014; 42:2118-2125.
16. Allardet al. J Neurochem 1987; 48:1553-1559.
17. Prajapati SC, Chauhan SS. FEBS J 2011; 278:3256-3276.
18. Ocaranza MP, Jalil JE. Hypertens (Dallas, Tex 1979) 2016; 68:552-554.
19. Rehfeld L, J Appl Lab Med 2019 JALM 3(6):943-953*.*
20. Deniau et al. Eur J Heart Fail 2020 Feb;22(2):290-299.
21. Levy J Am Coll Cardiol 2018; 72:173-182.
22. Kohsaka et al. Arch Intern Med 2005; 165:1643-1650.
23. Hochmanet al. N Engl J Med 1999; 341:625-634.
24. Thiele et al. N Engl J Med 2012; 367:1287-1296.
25. Thiele et al. N Engl J Med 2017; 377:2419-2432.
26. The TRIUMPH Investigators*, Alexander JH,et al. JAMA 2007; 297:1657.
27. Reyentovich et al. Nat Rev Cardiol 2016; 13:481-492.
28. Mebazaa et al. Intensive Care Med 2018; 44:760-773.
29. Bassi et al. Crit Care Res Pract 2013; 2013:654708.
30. Baran et al. Catheter Cardiovasc Interv 2019; 94:29-37.

## Claims

1. Inhibitor of the activity of DPP3 for use in a method of prevention or treatment of a refractory shock in a subject that either runs into shock or that has developed shock, wherein said subject has a level of DPP3 in a sample of bodily fluid of said subject that is above a predetermined threshold when determined by a method for predicting or diagnosing a refractory shock in a subject that either runs into shock or that has developed shock, wherein said method is comprising the steps:
• determining the level of DPP3 in a sample of bodily fluid of said subject, said sample of bodily fluid is selected from the group of whole blood, plasma, and serum.;
• comparing said level of determined DPP3 to a predetermined threshold that is between 25 and 150 ng/ml for plasma DPP3,
wherein said subject is predicted to run into refractory shock or is diagnosed as having refractory shock if said determined level of DPP3 is above said predetermined threshold and,
wherein the inhibitor of the activity of DPP3 is selected from the group comprising anti-DPP3 antibody or anti-DPP3 antibody fragment, and
wherein said anti-DPP3 antibody or anti-DPP3 antibody fragment is binding to an epitope according to SEQ ID NO.: 2, wherein said epitope is comprised in a DPP3 protein or functional derivative thereof and wherein said anti-DPP3 antibody or anti-DPP3 antibody fragment is a monoclonal antibody or a monoclonal antibody fragment thereof.

2. Inhibitor of the activity of DPP3 for use in a method of prevention or treatment of a refractory shock in a subject that either runs into shock or that has developed shock according to claim 1, wherein said anti-DPP3 antibody or the anti-DPP3 antibody fragment is a human or humanized antibody or derived therefrom or humanized antibody fragment or derived therefrom.

3. Inhibitor of the activity of DPP3 for use in a method of prevention or treatment of a refractory shock in a subject that either runs into shock or that has developed shock according to claim 1 or 2, wherein one or more (murine) CDR's are grafted into a human immunoglobulin backbone.

4. Inhibitor of the activity of DPP3 for use in a method of prevention or treatment of a refractory shock in a subject that either runs into shock or that has developed shock according to any of claims 1 to 3, a humanized monoclonal anti-DPP3 antibody or monoclonal anti-DPP3 antibody fragment thereof that is directed to and binding to an epitope according to SEQ ID NO.: 2, wherein said epitope is comprised in a DPP3 protein or a functional derivative thereof, and wherein the heavy chain comprises at least one CDR of:
SEQ ID NO.: 6, SEQ ID NO.: 7 or SEQ ID NO.: 8
and wherein the light chain comprises at least one CDR of:
SEQ ID NO.: 9, KVS or SEQ ID NO.: 10.

5. Inhibitor of the activity of DPP3 for use in a method of prevention or treatment of a refractory shock in a subject that either runs into shock or that has developed shock according to any of claims 1 to 4, wherein said humanized monoclonal anti-DPP3 antibody or monoclonal anti-DPP3 antibody fragment thereof that is directed to and binding to an epitope according to SEQ ID NO.: 2, wherein said epitope is comprised in a DPP3 protein or a functional derivative thereof, a wherein said inhibitor is a monoclonal antibody, wherein the complementarity determining regions (CDR's) in the heavy chain comprises the sequences: SEQ ID NO.: 6, SEQ ID NO.: 7 and/ or SEQ ID NO.: 8 and the complementarity determining regions (CDR's) in the light chain comprises the sequences: SEQ ID NO.: 9, KVS and/or SEQ ID NO.: 10.

6. Inhibitor of the activity of DPP3 for use in a method of prevention or treatment of a refractory shock in a subject that either runs into shock or that has developed shock according to any of claims 1 to 5, wherein wherein said shock is selected from the group comprising shock due to hypovolemia, cardiogenic shock, obstructive shock and distributive shock, in particular cardiogenic shock or septic shock.

7. Inhibitor of the activity of DPP3 for use in a method of prevention or treatment of a refractory shock in a subject that either runs into shock or that has developed shock according to any of claims 1 to 6, wherein said Inhibitor is administered in combination with an Angiotensin-Receptor-Agonist and/or precursor thereof , and wherein said Angiotensin-Receptor-Agonist and/ or precursor thereof that is selected from the group comprising angiotensin I, angiotensin II, angiotensin III, angiotensin IV, in particular angiotensin II and/or a precursor thereof wherein said precursor is a compound which is able to generate or to release an Angiotensin-Receptor-Agonist peptide under physiological conditions, in particular selected from the group comprising
- pegylated forms of Angiotensin-Receptor-Agonist peptides
- larger peptides comprising Angiotensin-Receptor-Agonist peptides, which upon selective cleaving form the Angiotensin-Receptor-Agonist peptide
- angiotensinogen,
- angiotensin I or its homologues,
- peptides with protected amino acids, e.g., having protecting groups at one or more amino groups, more particularly protecting groups selected from the group consisting of benzyloxycarbonyl, t-butyloxycarbonyl (BOC), fluorenylmethyloxycarbonyl (FMOC), formyl, acetyl, and acyl, and/or having protecting groups at one or more carboxylic acid groups, more particularly benzyl esters or t-butyl esters,
- precursor peptides having amino acid substitutions, deletions, additions, the substitutions and additions including the standard D and L amino acids and modified amino acids, such as, for example, amidated and acetylated amino acids, wherein the therapeutic activity of the base peptide sequence is maintained at a pharmacologically useful level.

8. Inhibitor of the activity of DPP3 for use in a method of prevention or treatment of a refractory shock in a subject that either runs into shock or that has developed shock according to claim 7, wherein said Angiotensin-Receptor-Agonist and/ or precursor thereof is selected from the group comprising angiotensin I, angiotensin II, angiotensin III, angiotensin IV, in particular angiotensin II.

9. Inhibitor of the activity of DPP3 for use in a method of prevention or treatment of a refractory shock in a subject that either runs into shock or that has developed shock according to claims 1 to 8, wherein in said therapy a treatment with vasopressors is withheld and/ or terminated if said determined level of DPP3 is above said predetermined threshold.

10. Inhibitor of the activity of DPP3 for use in a method of prevention or treatment of a refractory shock in a subject that either runs into shock or that has developed shock according to claims 1 to 9, wherein in addition in said therapy the level of Pro-adrenomedullin or fragments thereof is determined in a sample of bodily fluid of said subject and wherein treatment with an anti-ADM antibody or anti-ADM antibody fragment is initiated and/or continued when the level of Pro-adrenomedullin or fragments thereof in said sample is above a certain threshold and/or wherein a treatment with an anti-ADM antibody or anti-ADM antibody fragment is withheld and/ or terminated if the said determined level of Pro-adrenomedullin or fragments thereof is below said predetermined threshold.
